(19) Europäisches Patentamt
European Patent Office
Office européen des brevets

(11) Veröffentlichungsnummer: **0 354 522 A1**

# EUROPÄISCHE PATENTANMELDUNG

(21) Anmeldenummer: **89114601.1**

(51) Int. Cl.⁴: **A61K 37/64 , C07F 5/02**

(22) Anmeldetag: **08.08.89**

Patentansprüche für folgenden Vertragsstaat: ES + GR.

(30) Priorität: **12.08.88 DE 3827340**

(43) Veröffentlichungstag der Anmeldung: **14.02.90 Patentblatt 90/07**

(84) Benannte Vertragsstaaten: **AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Mölling, Karin, Prof. Dr.**
**Ihnestrasse 43**
**D-1000 Berlin 33(DE)**
Erfinder: **Kleemann, Heinz-Werner, Dr.**
**Jahnstrasse 6**
**D-6092 Kelsterbach(DE)**
Erfinder: **Urbach, Hansjörg, Dr.**
**Le Lavandoustrasse 41**
**D-6242 Kronberg/Taunus(DE)**
Erfinder: **König, Wolfgang, Dr.**
**Eppsteiner Strasse 25**
**D-6238 Hofheim am Taunus(DE)**
Erfinder: **Ruppert, Dieter, Dr.**
**Schreyerstrasse 30**
**D-6242 Kronberg/Taunus(DE)**
Erfinder: **Winkler, Irvin, Dr.**
**In den Eichen 40**
**D-6237 Liederbach(DE)**
Erfinder: **Paessens, Arnold, Dr.**
**Stresemannstrasse 56**
**D-5657 Haan(DE)**

(54) **Verwendung von alpha-Aminoboronsäure-Derivaten zur Prophylaxe und Behandlung von Viruserkrankungen.**

(57) Die Erfindung betrifft die Verwendung von Verbindungen der Formel I

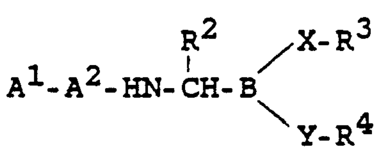

(I)

worin $A^1$ einen der Rest der Formeln

EP 0 354 522 A1

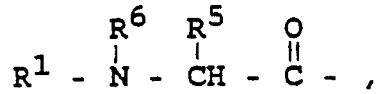

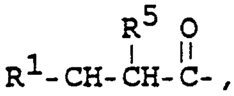, 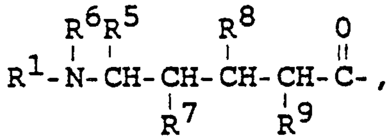, 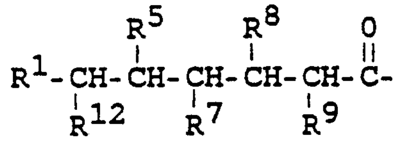 oder

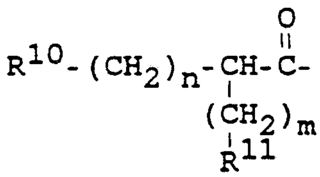

bedeutet,
$A^2$ abwesend ist oder für einen Rest der Formel

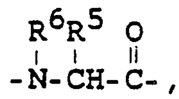

steht, $R^2$, $R^3$ und $R^4$ wie in der Beschreibung definiert sind und X und Y unabhängig voneinander für -O- oder $-NR^{13}$ stehen, sowie deren Salze zur Therapie und Prophylaxe von Viruserkrankungen, insbesondere von Erkrankungen, die durch das HIV verursacht werden.

# Verwendung von α-Aminoboronsäure-Derivaten zur Prophylaxe und Behandlung von Viruserkrankungen

Die vorliegende Erfindung betrifft die Verwendung von α-Aminoboronsäure-Derivaten zur Prophylaxe und Behandlung von Viruserkrankungen.

Seitdem die Gefährdung des Menschen durch HIV (Human Immune Deficiency Virus)-Infektionen bekannt geworden ist, wird intensiv nach chemotherapeutischen Methoden zur Bekämpfung von Viruserkrankungen gearbeitet. Es sind bereits verschiedene chemotherapeutische Mittel gegen Viruserkrankungen entwickelt werden, wie z.B. das Acyclovir gegen Herpes-Viren. Gegen HIV sind ebenfalls bereits Chemotherapeutika entwickelt worden (z.B. das Azidothymidin), die aber größtenteils insbesondere wegen ihrer Nebenwirkungen noch nicht befriedigend sind.

Überraschenderweise wurde nun gefunden, daß α-Aminoboronsäure-Derivate gegen HIV wirksam sind. Die medizinische Wirksamkeit der α-Aminoboronsäure-Derivate als Renin-Hemmer wurde bereits in der europäischen Patentanmeldung 0 315 574 beschrieben. Die α-Aminoboronsäure-Derivate sind bei der Bekämpfung des HIV besonders wertvoll, weil sie ein neues Konzept bei HIV-Bekämpfung ermöglichen. Während viele Chemotherapeutika gegen HIV wirksam sind weil sie die reverse Transkriptase hemmen, beruht die Wirkung der α-Aminoboronsäure-Derivate darauf, daß eine Virus-Protease gehemmt wird. Z.B. ist am 5′-Ende des HIV-POL-Gens eine Protease codiert [Proc. Nat. Acad. Sci. USA 84, 8903 (1987)], die sich selbst aus dem GAG-POL-Polypeptidvorläufer herausschneidet und anschließend das GAG-Polyprotein (Kernproteine) prozessiert. Die zuletzt genannte Protease wird u.a. durch die α-Aminoboronsäure-Derivate wirksam inhibiert.

Die Erfindung betrifft demzufolge die Verwendung von Verbindungen der Formel I

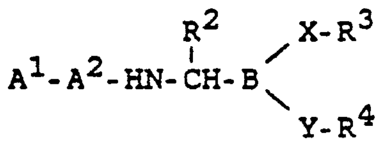

(I)

in welcher

A' einen Rest der Formeln II, III, IV, V oder VI bedeutet

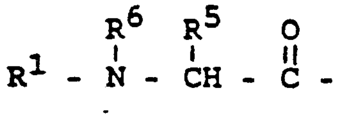

(II)

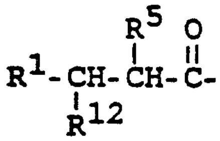

(III)

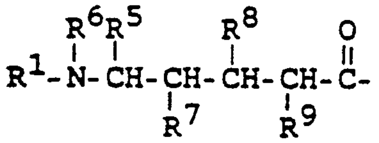

(IV)

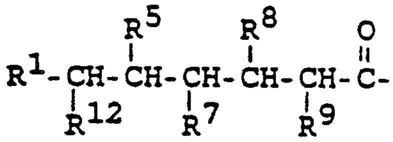

(V)

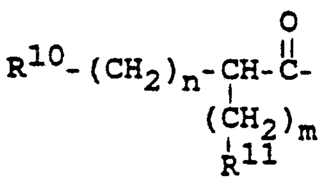

(VI)

worin

$R^1$ $a_1$) Wasserstoff, $(C_1-C_{12})$-Alkyl, das gegebenenfalls ein-oder zweifach ungesättigt ist und das gegebenenfalls durch bis zu 3 gleiche oder verschiedene Reste aus der Reihe Hydroxy, $(C_1-C_7)$-Alkoxy, $(C_1-C_8)$-Alkanoyloxy, Carboxy, $(C_1-C_7)$-Alkoxycarbonyl, F, Cl, Br, I, Amino, Carbamoyl, Amidino, das gegebenenfalls durch einen, zwei oder drei $(C_1-C_8)$-Alkylreste substituiert sein kann, Guanidino, das gegebenenfalls durch einen, zwei, drei oder vier $(C_1-C_8)$-Alkylreste substituiert sein kann, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethoxycarbonylamino substituiert ist, mono-, bi- oder tricyclisches $(C_3-C_{18})$-Cycloalkyl, $(C_3-C_{18})$-Cycloalkyl-$(C_1-C_6)$-alkyl oder $(C_6-C_{14})$-Aryl, das gegebenenfalls durch einen oder zwei gleiche oder verschiedene Reste aus derReihe F, Cl, Br, I, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogenen substituiertes Anilino und Trifluormethyl substituiert ist, $(C_6-C_{14})$-Aryl-$(C_1-C_6)$-alkyl, worin der Arylteil gegebenenfalls durch einen oder zwei gleiche oder verschiedenen Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylanino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidino-$(C_1-C_8)$-alkyl substituiert ist, oder für den Rest eines 5- oder 6-gliedrigen monocyclischen oder 9-oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht, und der gegebenenfalls wie $(C_6-C_{14})$-Aryl unter $a_1$) definiert, mono-, di- oder trisubstituiert ist, bedeutet

oder

$a_2$) einen Rest der Formel VII bedeutet

$R^1$ - W , (VII)

worin $R^1$ wie $R^1$ unter $a_1$) definiert ist und W für -CO-, -O-CO-, $-SO_2-$, -SO-, $-HN-SO_2-$, -HN-CO-, -CH(OH)- oder -N(OH)- steht,

$R^2$, $R^5$ und $R^9$ unabhängig voneinander wie $R^1$ unter $a_1$) definiert sind,

$R^3$ und $R^4$ unabhängig voneinander Wasserstoff oder $(C_1-C_{12})$-Alkyl bedeuten, das gegebenenfalls ein- bis zweifach ungesättigt ist und das gegebenenfalls durch bis zu 3 gleiche oder verschiedene Reste aus der Reihe Hydroxy, $(C_1-C_7)$-Alkoxy, Amino, Mono- oder Di-$(C_1-C_7)$-alkylamino substituiert ist, oder zusammen mit Bor, X und Y ein mono-, bi- oder tricyclisches, gesättigtes oder teilweise ungesättigtes mono-, di-, tri- oder tetra-$(C_1-C_{12})$-alkyliertes Ringsystem mit 5-18 Ringgliedern, das außer Bor, X, Y und Kohlenstoff noch ein -O- Glied, ein -$NR^{13}$-Glied oder ein -$CR^{14}R^{15}$-Glied enthalten kann, bilden,

X und Y unabhängig voneinander für -O- oder -$NR^{13}$- stehen, $R^6$ für Wasserstoff oder $(C_1-C_8)$-Alkyl steht, oder zusammen mit $R^5$ ein mono- oder bicyclisches, gesättigtes oder teilweise ungesättigtes Ringsystem mit 5-12 Ringgliedern bildet, $R^7$ und $R^8$ unabhängig voneinander Wasserstoff, Hydroxy, Amino, Fluor, Amino-$(C_1-C_4)$-alkyl, Hydroxy-$(C_1-C_4)$-alkyl, $(C_1-C_4)$-Alkyl, das auch einfach ungesättigt sein kann, bedeuten,

$R^{10}$ und $R^{11}$ unabhängig voneinander Wasserstoff, Hydroxy oder $(C_6-C_{14})$-Aryl bedeuten, das gegebenenfalls durch einen oder zwei gleiche oder verschiedene Reste aus der Reihe F, Cl, Br, I, Hydroxy, $(C_1-C_7)$-Alkoxy, $(C_1-C_7)$-Alkyl, $C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidino-$(C_1-C_8)$-alkyl substituiert ist, oder für den Rest eines 5-oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und oder 1 - 2 S-Atomen und oder 1 - 2 O-Atomen als Ringglieder stehen, der gegebenenfalls wie oben $(C_6-C_{14})$-Aryl mono- oder disubstituiert ist,

n und m unabhängig voneinander 0, 1, 2, 3 und 4 sein können,

$R^{12}$ Wasserstoff oder $(C_1-C_8)$-Alkyl ist, oder gemeinsam mit $R^1$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,

$A^2$ entweder abwesend ist, wobei gleichzeitig $A^1$ nicht für einen Rest der Formel II steht, oder einen N-terminal mit $A^1$ und C-terminal mit dem Aminoboronsäurederivat verknüpften Rest der Formel VIII bedeutet,

$$-\overset{\displaystyle R^6}{\overset{|}{N}}-\overset{\displaystyle R^5}{\overset{|}{CH}}-\overset{\displaystyle O}{\overset{\|}{C}}- \qquad (VIII)$$

wobei $R^5$ und $R^6$ wie oben definiert sind,

$R^{13}$ Wasserstoff oder $(C_1-C_{12})$-Alkyl, das gegebenenfalls ein- bis zweifach ungesättigt ist und das gegebenenfalls durch bis zu 3 gleiche oder verschiedene Reste aus der Reihe Hydroxy, $(C_1-C_7)$-Alkoxy, Amino, Mono- oder Di-$(C_1-C_7)$-alkylamino substituiert ist, bedeutet

$R^{14}$ und $R^{15}$ unabhängig voneinander Wasserstoff, $(C_1-C_8)$-Alkyl, Hydroxymethyl, 2-Hydroxyethyl, (3-Hydroxysulfonyl,2-hydroxypropyl)amino, (2-Hydroxysulfonylethyl)amino, (2-Hydroxysulfonylpropyl)amino, (Carboxymethyl)amino, Bis(2-hydroxyethyl)amino bedeuten sowie deren physiologisch verträglichen Salzen, zur Therapie und Prophylaxe von Viruserkrankungen, insbesondere von Erkrankungen, die durch das HIV verursacht werden.

Unter einem Rest eines 5- oder 6-gliedrigen monocyclischen oder 9- oder 10-gliedrigen bicyclischen Heteroaromaten mit mindestens 1 C-Atom, 1 - 4 N-Atomen und/oder 1 - 2 S-Atomen und/oder 1 - 2 O-Atomen als Ringglieder werden Reste von Heteroaromaten verstanden, wie sie beispielsweise in Katritzky, Lagowski, Chemie der Heterocyclen, Berlin, Heidelberg 1968 definiert sind. Der Heteroaromaten-Rest kann durch einen, zwei oder drei, vorzugsweise einen oder zwei gleiche oder verschiedene Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino oder Trifluormethyl substituiert sein. Monocyclische Heteroaromaten sind z.B. Thiophen, Furan, Pyrrol, Imidazol, Pyrazol, Pyridin, Pyrazin, Pyrimidin, Pyridazin, 1,2,4-Triazol, Thiazol, Tetrazol, Isothiazol, Oxazol und Isoxazol. Bicyclische Heteroaromaten sind z.B. Benzothiophen, Benzofuran, Indol, Isoindol, Indazol, Benzimidazol, Chinolin, Isochinolin, Phthalazin, Chinoxalin, Chinazolin und Cinnolin. Entsprechendes gilt für von Heteroaryl abgeleitete Reste, wie z.B. ganz oder teilweise hydriertes Heteroaryl, u.a. auch z.B. Benzodioxolan, Heteroaryloxy, Heteroarylthio und Heteroaryl-alkyl. Alkyl kann geradkettig oder verzweigt sein. Entsprechendes gilt für davon abgeleitete Reste, wie z.B. Alkoxy, Alkylthio, Alkylamino, Dialkylamino, Alkylsulfinyl, Alkylsulfonyl, Alkanoyl und Aralkyl.

$(C_6-C_{14})$-Aryl ist beispielsweise Phenyl, Naphthyl, Biphenylyl oder Fluorenyl; bevorzugt ist Phenyl. Entsprechendes gilt für davon abgeleitete Reste, wie z.B. Aryloxy, Aroyl, Aralkyl und Aralkyloxy. Unter Aralkyl versteht man einen mit $(C_1-C_6)$-Alkyl verknüpften unsubstituierten oder substituierten $(C_6-C_{14})$-Aryl-

Rest, wie z.B. Benzyl, α- und β-Naphthylmethyl, Halobenzyl und Alkoxybenzyl, wobei Aralkyl jedoch nicht auf die genannten Reste beschränkt wäre.

Unter Salzen von Verbindungen der Formel I sind insbesondere pharmazeutisch verwendbare oder nicht-toxische Salze zu verstehen.

Solche Salze werden beispielsweise von Verbindungen der Formel I, welche saure Gruppen, z.B. Carboxy, enthalten, mit Alkali- oder Erdalkalimetallen gebildet, wie Na, K, Mg und Ca, sowie mit physiologisch verträglichen organischen Aminen, wie z.B. Triethylamin und Tri-(2-hydroxy-ethyl)amin.

Verbindungen der Formel I, welche basische Gruppen, z.B. eine Aminogruppe oder eine Guanidinogruppe, enthalten, bilden Salze mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure.

Bevorzugt ist die erfindungsgemäße Verwendung von Verbindungen der Formel I, in denen die Reste wie folgt definiert sind:

$A^1$ ist wie auf der Seite 2 definiert,

$R^1$ bedeutet vorzugsweise Wasserstoff oder steht für ($C_1$-$C_{10}$-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, 2-Pyridyl-($C_1$-$C_8$)-alkyl, 3-Pyridyl-($C_1$-$C_8$)-alkyl, 4-Pyridyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl, ($C_1$-$C_4$)-Alkoxycarbonyl-($C_1$-$C_{10}$)-alkyl, ($C_1$-$C_8$)-Alkylsulfonyl, ($C_1$-$C_8$)-Alkylsulfinyl, Hydroxy-($C_1$-$C_{10}$)-alkanoyl, wie 2-Hydroxypropionyl oder 2-Hydroxy-3-methylbutyryl, ($C_1$-$C_8$)-Alkanoyloxy-($C_1$-$C_{10}$)-alkyl, ($C_1$-$C_{11}$)-Alkanoyl, wie n-Decanoyl, Formyl, Acetyl, Pivaloyl, Isovaleryl oder Isobutyryl, gegebenenfalls geschütztes Amino-($C_1$-$C_{11}$)-alkanoyl, wie 4-Aminobutyryl, 5-Aminopentanoyl, 6-Aminohexanoyl, 4-N-tert.-Butoxycarbonylaminobutyryl, 5-N-tert.-Butoxycarbonylaminopentanoyl oder 6-N-tert.-Butoxycarbonylaminohexanoyl, Di-($C_1$-$C_7$)-alkylamino-($C_2$-$C_{11}$)-alkanoyl, wie Dimethylaminoacetyl, ($C_3$-$C_9$)-Cycloalkylcarbonyl, wie Cyclopropylcarbonyl, Cyclobutylcarbonyl, Cyclopentylcarbonyl oder Cyclohexylcarbonyl, ($C_6$-$C_{10}$)-Aryl-($C_2$-$C_{11}$)-alkanoyl, wie Phenylacetyl, Phenylpropanoyl oder Phenylbutanoyl, 2-Pyridyl-($C_1$-$C_8$)-alkanoyl, 3-Pyridyl-($C_1$-$C_8$)-alkanoyl, 4-Pyridyl-($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl, ($C_1$-$C_{10}$)-Alkoxycarbonyl, wie Methoxycarbonyl, Ethoxycarbonyl oder tert.-Butoxycarbonyl, substituiertes ($C_1$-$C_{10}$)-Alkoxycarbonyl, wie 2-(Trimethylsilyl)ethoxycarbonyl, 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, wie Benzyloxycarbonyl oder 9-Fluorenylmethoxycarbonyl, oder $R^1$ bildet bevorzugt gemeinsam mit $R^{12}$ ein mono- oder bicyclisches, gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxydiert sein kann,

$R^2$ ist vorzugsweise ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, Dithiolanyl, Dithiolanylmethyl, Dithiolanylethyl, Dithianyl, Dithianylmethyl oder Dithianylethyl,

$R^3$ und $R^4$ sind vorzugsweise unabhängig voneinander Wasserstoff, ($C_1$-$C_{12}$)-Alkyl oder bilden zusammen mit Bor, X und Y ein mono-, bi- oder tricyclisches, gesättigtes oder teilweise ungesättigtes, gegebenenfalls mono-, di-, tri- oder tetra-($C_1$-$C_{12}$)-alkyliertes Ringsystem mit 5-18 Ringgliedern, das außer Bor, X, Y und Kohlenstoff noch ein -O- Glied, ein -$NR^{13}$-Glied oder ein -$CR^{14}R^{15}$-Glied enthalten kann,

X und Y sind unabhängig voneinander bevorzugt -O- oder -$NR^{13}$-,

$R^5$ und $R^9$ sind unabhängig voneinander bevorzugt ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Carbamoyl, Amidino, Guanidino, ($C_1$-$C_5$)-Alkoxycarbonylamino, ($C_7$-$C_{15}$)-Aralkoxycarbonylamino und 9-Fluorenylmethoxycarbonylamino substituiert ist, ($C_3$-$C_{12}$)-Cycloalkyl-($C_1$-$C_3$)-alkyl, mono- oder bicyclisches ($C_6$-$C_{14}$)-Aryl-($C_1$-$C_3$)Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist oder stehen bevorzugt für ($C_1$-$C_3$)-Alkyl, substituiert mit dem Rest eines 5- oder 6-gliedrigen monocyclischen oder 9- oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie auf Seite 4 für ($C_6$-$C_{14}$)-Aryl beschrieben mono- oder disubstituiert ist,

$R^6$ ist bevorzugt Wasserstoff, Methyl oder Ethyl oder bildet zusammen mit $R^5$ bevorzugt Pyrrolidin oder Piperidin, die jeweils noch mit Cyclopentyl, Cyclohexyl oder Phenyl anneliert sein können,

$R^7$ und $R^8$ sind unabhängig voneinander bevorzugt Wasserstoff, Hydroxy, Amino, Fluor, Hydroxymethyl oder Aminomethyl,

$R^{10}$ und $R^{11}$ sind unabhängig voneinander bevorzugt Wasserstoff, Hydroxy, Phenyl, 2- oder 3-Thienyl, 2-, 3- oder 4-Pyridyl, 1-, 2- oder 4-Imidazolyl, 1- oder 2-Naphthyl; 2- oder 3-Benzo[b]thienyl,
n und m können unabhängig voneinander 0, 1, 2, 3 und 4 bedeuten,
$R^{12}$ ist wie auf Seite 5 definiert,
$R^{13}$ ist bevorzugt Wasserstoff oder ($C_1$-$C_{12}$)-Alkyl,
$R^{14}$ und $R^{15}$ sind wie auf Seite 6 definiert,
$A^2$ ist abwesend, wobei gleichzeitig $A^1$ nicht für einen Rest der Formel II steht, oder bedeutet einen Rest der Formel VIII, wobei $R^5$ und $R^6$ wie auf den Seiten 9 und 10 definiert sind.

Besonders bevorzugt ist die erfindungsgemäße Verwendung von Verbindungen der Formel I, in denen die Reste wie folgt definiert sind:
$A^1$ ist wie auf der Seite 2 definiert,
$R^1$ bedeutet besonders bevorzugt ($C_1$-$C_8$)-Alkylsulfonyl, ($C_1$-$C_8$)-Alkylsulfinyl, Hydroxy-($C_1$-$C_{10}$)alkanoyl, wie 2-Hydroxypropionyl oder 2-Hydroxy-3-methylbutyryl, ($C_1$-$C_8$)-Alkanoyloxy-($C_1$-$C_{10}$)-alkyl, ($C_1$-$C_{11}$)Alkanoyl, wie n-Decanoyl, Formyl, Acetyl, Pivaloyl, Isovaleryl oder Isobutyryl, Amino-($C_1$-$C_{11}$)-alkanoyl, wie 4-Aminobutyryl, 5-Aminopentanoyl, 6-Aminohexanoyl, Di-($C_1$-$C_7$)-alkylamino-($C_2$-$C_{11}$)-alkanoyl, wie Dimethylaminoacetyl, ($C_3$-$C_9$)-Cycloalkylcarbonyl, wie Cyclopropylcarbonyl, Cyclobutylcarbonyl, Cyclopentylcarbonyl oder Cyclohexylcarbonyl, ($C_6$-$C_{10}$)-Aryl-($C_2$-$C_{11}$) alkanoyl, wie Phenylacetyl, Phenylpropanoyl oder Phenylbutanoyl, 2-Pyridyl-($C_1$-$C_8$)-alkanoyl, 3-Pyridyl-($C_1$-$C_8$)-alkanoyl, 4-Pyridyl-($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, wie 4-Chlorbenzoyl, 4-Methylbenzoyl, 2-Methoxycarbonylbenzoyl oder 4-Methoxybenzoyl, Pyrrolyl-2-carbonyl, Pyridyl-3-carbonyl, Benzolsulfonyl, ($C_1$-$C_{10}$)-Alkoxycarbonyl, wie Methoxycarbonyl, Ethoxycarbonyl oder tert.-Butoxycarbonyl, substituiertes ($C_1$-$C_{10}$)-Alkoxycarbonyl, wie 2-(Trimethylsilyl)ethoxycarbonyl, 2,2,2-Trichlorethoxycarbonyl oder 1,1-Dimethyl-2,2,2-trichlorethoxycarbonyl, ($C_6$-$C_{14}$)-Aryl-($C_1$-$C_6$)-alkoxycarbonyl, wie Benzyloxycarbonyl oder 9-Fluorenylmethoxycarbonyl,
$R^2$ ist besonders bevorzugt ($C_3$-$C_{12}$)-Alkyl, mono-, bi- oder tricyclisches ($C_3$-$C_{18}$)-Cycloalkyl oder ($C_3$-$C_{18}$)-Cycloalkylmethyl, wobei der Cycloalkylteil gegebenenfalls durch ($C_1$-$C_4$)-Alkyl substituiert ist; ($C_6$-$C_{14}$)-Arylmethyl; Dithiolanyl, Dithiolanylmethyl, Dithianyl und Dithianylmethyl,
$R^3$ und $R^4$ sind besonders bevorzugt Wasserstoff oder bilden gemeinsam mit Bor, X und Y ein mono-, bi- oder tricyclisches mono-, di-, tri- oder tetra-($C_1$-$C_{12}$)-alkyliertes, gesättigtes oder teilweise ungesättigtes Ringsystem mit 5-18 Ringgliedern, das außer Bor, X, Y und Kohlenstoff noch ein -O- Glied, ein -$NR^{13}$-Glied oder ein -$CR^{14}R^{15}$-Glied enthalten kann,
X und Y stehen besonders bevorzugt für -O-,
$R^5$ und $R^8$ sind unabhängig voneinander besonders bevorzugt Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto, 1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-thienylmethyl, 2-(2-Thienyl)ethyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methylimidazol-4-yl)methyl, (3-Methyl-Imidazol-4-yl)methyl, Phenyl, 1-Naphthylmethyl, 2-Naphthylmethyl, 2-Phenylethyl, 2-Thiazolylmethyl, 4-Thiazolylmethyl, 3-Pyrazolylmethyl, 4-Pyrimidinylmethyl, Indol-2-ylmethyl, 2-Benzo[b]thienylmethyl, 3-Benzo[b]thienylmethyl, 2-Furylmethyl, Cyclopentyl
$R^6$ bedeutet besonders bevorzugt Wasserstoff oder Methyl oder bildet gemeinsam mit $R^5$, und der diese Reste tragenden -N-CH-Gruppe ein Tetrahydroisochinolinoder Azabicyclooctan-Gerüst,
einer der Reste $R^7$ oder $R^8$ bedeutet besonders bevorzugt Wasserstoff, der jeweils andere Rest Hydroxy, Amino, Fluor, Hydroxymethyl oder Aminomethyl,
$R^{10}$ und $R^{11}$ sind unabhängig voneinander besonders bevorzugt Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl,
n und m sind unabhängig voneinander besonders bevorzugt 0, 1 oder 2,
$R^{12}$ ist besonders bevorzugt Wasserstoff oder Methyl, oder bildet gemeinsam mit $R^1$ ein mono- oder bicyclisches gesättigtes oder teilweise ungesättigtes Ringsystem mit 5-12 Ringgliedern, das außer Kohlenstoff noch 1 Schwefelatom enthalten kann, welches gegebenenfalls zum Sulfoxid oder Sulfon oxidiert sein kann,
$R^{13}$ ist besonders bevorzugt Wasserstoff oder ($C_1$-$C_4$)-Alkyl, $R^{14}$ und $R^{15}$ sind wie auf Seite 6 definiert,
$A^2$ ist abwesend, wobei gleichzeitig $A^1$ nicht für einen Rest der Formel II steht, oder bedeutet einen Rest der Formel VIII, wobei $R^5$ und $R^6$ wie auf der Seite 4 definiert ist.

Ferner ist besonders bevorzugt die Verwendung von Verbindungen der Formel I, in denen

$A^1$ für einen Rest der Formel II, III oder VI steht, worin $R^1$ $(C_1-C_6)$-Alkylsulfonyl, wie z.B. Ethylsulfonyl, Isopropylsulfonyl, Isobutylsulfonyl, sec.-Butylsulfonyl oder tert.-Butylsulfonyl; $(C_1-C_6)$-Alkylsulfinyl, wie z.B. Ethylsulfinyl, Isopropylsulfinyl, Isobutylsulfinyl, sec.-Butylsulfinyl oder tert.-Butylsulfinyl; $(C_1-C_6)$-Alkanoyl, wie z.B. Acetyl, Propionyl, Isobutyryl, Isovaleryl oder Pivaloyl;

Amino-$(C_1-C_6)$-alkanoyl, wie z.B. (3-Amino,3,3-dimethyl)propionyl; $(C_1-C_6)$-Alkoxycarbonyl, wie z.B. Ethoxycarbonyl, Isobutoxycarbonyl oder tert.-Butoxycarbonyl; $(C_6-C_{10})$-Aryl-$(C_1-C_6)$-alkoxycarbonyl, wie z.B. Benzyloxycarbonyl, 1- oder 2-Naphthylmethoxycarbonyl bedeutet,

$R^2$ $(C_5-C_8)$-Cycloalkyl; $(C_5-C_{11})$-Cycloalkylmethyl; $[(C_1-C_4)$-Alkyl-cyclohexyl]methyl; $(C_3-C_4)$-Alkyl, das durch $(C_1-C_3)$-Alkyl substituiert ist; $(C_6-C_{10})$-Arylmethyl oder Dithiolan-2-yl-methyl bedeutet,

$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2'-imino-diethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,

X und Y bevorzugt für -O- stehen,

$R^5$ Wasserstoff, n-Propyl, Isopropyl, n-Butyl, Isobutyl, Cyclopentyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,

$R^6$ für Wasserstoff oder Methyl steht,

$R^{10}$ und $R^{11}$ unabhängig voneinander 1-Naphthyl oder 2-Thienyl bedeuten,

n und m für 1 stehen,

$R^{12}$ Wasserstoff bedeutet,

$R^{14}$ und $R^{15}$ unabhängig voneinander Wasserstoff, $(C_1-C_8)$-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)amino, (Carboxymethyl)amino oder Bis(2-hydroxyethyl)amino bedeuten,

und

$A^2$ für einen Rest der Formel VIII steht, wobei $R^5$ und $R^6$ wie auf den Seiten 12 und 13 definiert sind.

Die Verbindungen der Formel I lassen sich herstellen, indem man ein Fragment mit endständiger Carboxylgruppe oder dessen reaktives Derivat mit einem entsprechenden Fragment mit freier Aminogruppe kuppelt, gegebenenfalls zum Schutz weiterer funktioneller Gruppen (eine) temporär eingeführte Schutzgruppe(n) abspaltet und die so erhaltene Verbindung gegebenenfalls in ihr verträgliches Salz überführt.

Fragmente einer Verbindung der Formel I mit einer endständigen Carboxylgruppe besitzen die nachstehenden Formeln IX bis X

$A^1$ - OH (IX)

$A^1$ - $A^2$ - OH (X)

Fragmente einer Verbindung der Formel I mit einer endständigen Aminogruppe besitzen die nachstehenden Formeln XI bis XIV

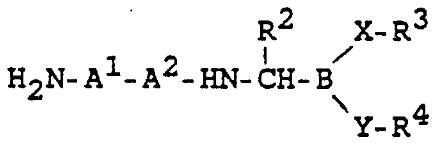

(XI)

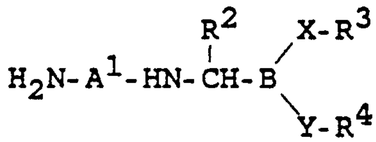

(XII)

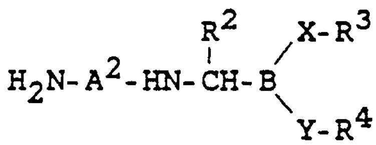

(XIII)

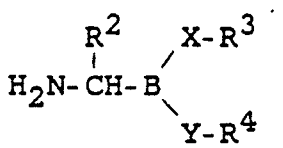

(XIV)

Methoden, die zur herstellung einer Amidbindung geeignet sind, werden z.B. in Houben-Weyl, Methoden der organischen Chemie, Band 15/2; Bodanszky et al., Peptide Synthesis, 2nd ed. (Wiley & Sons, New York 1976) oder Gross, Meienhofer, The Peptides: Analysis, synthesis, biology (Academic Press, New York 1979) beschrieben. Vorzugsweise werden die folgenden Methoden herangezogen:
Aktivestermethode mit N-Hydroxy-succinimid, 1-Hydroxybenzotriazol oder 3-Hydroxy-4-oxo-3,4-dihydro-1,2,3-benzotriazin als Alkoholkomponente, Kupplung mit einem Carbodiimid wie Dicyclohexylcarbodiimid oder mit Propanphosphonsäureanhydrid und die Gemischt-Anhydrid-Methode mit Pivaloylchlorid oder Chlorameisensäureethylester oder -isobutylester.

Fragmente der Formel IX, sofern sie unter

a) Formel II fallen, werden nach den allgemein bekannten Methoden zur Herstellung von Aminosäuren synthetisiert;

b) Formel III fallen, werden ausgehend von den entsprechenden Aminosäuren synthetisiert, wobei deren Chiralitätszentrum erhalten bleibt. Diazotierung bei -20 °C bis 50 °C in verd. Mineralsäuren führt zu α-Bromcarbonsäuren oder über die Milchsäuren zu α-Trifluormethansulfonyloxy-Carbonsäuren, die mit einem $R^{1}$ und $R^{12}$ tragenden Nucleophil umgesetzt werden können;

c) Formel IV fallen, werden ausgehend von Aminoaldehyden, die nach B. Castro et al. (Synthesis 1983, 676) hergestellt werden, synthetisiert. Wittig-Reaktion mit Methyltriphenylphosphoniumbromid unter den allgemein bekannten Bedingungen und anschließende Epoxidierung mit m-Chlorperbenzoesäure liefert N-geschützte Aminoepoxide. Öffnung mit Trimethylsilylchlorid/NaI in Acetonitril im Temperaturbereich von -20 °C bis zum Siedepunkt des Lösungsmittels, Desilylierung mit CsF und Acetonierung mit 2,2-Dimethoxypropan/p-Toluolsulfonsäure bei 0 °C bis 110 °C liefert ein geschütztes Jodid. Dieses wird entweder direkt mit Esterenolaten nach den allgemein bekannten Bedingungen der C-Alkylierung von Esterenolaten umgesetzt oder, wenn höhere Reaktivität gewünscht ist, über das Formiat (hergestellt mit Na-Formiat in einem dipolar aprotischen Lösungsmittel bei 60 °C bis 150 °C) und den Alkohol in das Trifluormethansulfonat überführt. Mit diesem Elektrophil lassen sich auch Esterenolate umsetzen, die aufgrund sterischer Hinderung in $R^9$ sich nicht wie oben beschrieben mit dem Jodid umsetzen lassen;

d) Formel V fallen, werden ausgehend von der entsprechenden Carbonsäure durch Umsetzung mit einem Rest der Formel III hergestellt und nach der Überführung in die Aldehyde wie unter c) beschrieben weiter umgesetzt;

e) Formel VI fallen, werden ausgehend von Malonestern hergestellt, deren Alkylierung mit Arylalkylhalogeniden mono- oder disubstituierte Malonester liefert, die nach Verseifung durch Decarboxylierung in

die gewünschten Derivate überführt werden. Für den Fall, daß einer der Reste $R^{10}$ oder $R^{11}$ Hydroxy bedeutet, geht man von der entsprechenden Aminosäure aus und erhält nach Diazotierung (wie oben beschrieben) die Milchsäure (Anzahl der $R^{10}$ oder $R^{11}$ tragenden $CH_2$-Gruppen = 0) oder man geht von der substituierten Malonsäure aus, wobei Monoverseifung und selektive Reduktion (mit z.B. Diboran oder $LiAlH_4$) die 2-substituierte 3-Hydroxy-propionsäure ergibt.

Die Aminoboronsäurederivate aus Formel I werden ausgehend von Borsäuretrialkylestern wie z.B. $B(OMe)_3$, $B(OEt)_3$, $B(OiPr)_3$, $B(OnBu)_3$ oder $B(OtBu)_3$ hergestellt. Die Einführung von $R^2$ erfolgt über eine Grignard-Reaktion bei Temperaturen zwischen -100 °C und +50 °C, bevorzugt zwischen -78 °C und 0 °C in einem gegenüber metallorganischen Reagentien inerten Lösungsmittel wie Ethern, so zum Beispiel Diethylether, Tetrahydrofuran oder Dimethoxyethan. Unter gleichen Reaktionsbedingungen können auch entsprechende Alkyllithium- oder Aryllithiumverbindungen zur Einführung von $R^2$ dienen. Anschließend erfolgt zweckmäßigerweise die Überführung in einen relativ hydrolysebeständigen Boronsäureester wie den Pinakolester oder, wenn die Synthese asymmetrisch gestaltet werden soll in den Pinandiolester gewünschter Stereochemie oder den 1,2-Diisopropylethandiol-Ester. Dies erfolgt am Besten unter Säurekatalyse und Entfernung des Niederalkyl-Alkohols unter vermindertem Druck. Anschließend werden die Boronsäureester mit $LiCHCl_2$ bei -120 °C bis -70 °C, bevorzugt bei -110 °C bis -90 °C in einem Lösungsmittel wie Diethylether oder Tetrahydrofuran umgesetzt. Zunächst entsteht das Dichlormethylboronat, das schließlich bei Raumtemperatur unter $Cl^-$ -Eliminierung in den um eine (-CHCl-)-Gruppe verlängerten Boronsäureester umlagert. Zur Erhöhung der Reaktivität wird Chlor noch durch Jod substituiert durch Umsetzung mit NaI in Acetonitril bei 0 °C bis 50 °C. Dessen Umsetzung mit Lithium-bis-(trimethylsilyl)amid bei 0 °C bis 50 °C in einem gegenüber starken Basen inerten Lösungsmittel wie Ethern und anschließende Desilylierung des Produkts in Trifluoressigsäure liefert die substituierten α-Aminoboronsäureester als kristallisierende Trifluoressigsäuresalze. Diese können analog der Peptidkupplung mit Aminocarbonsäureestern mit den Resten $A^1$-OH oder $A^1$-$A^2$-OH umgesetzt werden.

Die zur Herstellung von Verbindungen der Formel I erforderlichen Vor- und Nachoperationen wie Einführung und Abspaltung von Schutzgruppen sind literaturbekannt und sind z.B. in T.W. Greene "Protective Groups in Organic Synthesis" (John Wiley & Sons, New York, 1981) beschrieben. Salze von Verbindungen der Formel I mit salzbildenden Gruppen werden in an sich bekannter Weise hergestellt, indem man z.B. eine Verbindung der Formel I mit einer basischen Gruppe mit einer stöchiometrischen Menge einer geeigneten Säure umsetzt. Stereoisomerengemische, insbesondere Diastereomerengemische, die gegebenenfalls bei der Synthese von Verbindungen der Formel I anfallen, können in an sich bekannter Weise durch fraktionierte Kristallisation oder durch Chromatographie getrennt werden.

In biologischen Tests zeigte sich, daß die erfindungsgemäßen Verbindungen enzyminhibitorische Wirkung haben und auch virale Enzyme wie die HIV-Protease hemmen. Besondere Bedeutung hat die HIV-Protease inhibierende Wirkung, die die erfindungsgemäßen Verbindungen insbesondere zur Therapie und Prophylaxe von durch Infektion mit HIV bedingten Erkrankungen qualifiziert. Die erfindungsgemäßen Verbindungen der allgemeinen Formel I zeigen in verwendeten in-vitro-Tests Hemmwirkungen bei Konzentrationen von etwa $10^{-4}$ bis $10^{-5}$ Mol/l.

Zum Gegenstand der Erfindung gehören weiterhin die Verwendung von Verbindungen der Formel I zur Herstellung eines Arzneimittels zur Therapie oder Prophylaxe von Viruserkrankungen, insbesondere von Erkrankungen, die durch das HIV verursacht werden sowie die genannten Arzneimittel.

Die Arzneimittel enthalten eine wirksame Menge mindestens eines Wirkstoffs der Formel I gegebenenfalls zusammen mit einem anorganischen oder organischen pharmazeutisch verwendbaren Trägerstoff.

Die erfindungsgemäßen Verbindungen können auf unterschiedliche Weise appliziert werden, z.B. nasal, oral, intravenös, intraduodenal oder subkutan. Vorzuziehen ist die orale oder die intravenöse Applikation. Bei oraler Verabreichung ist eine Dosis von etwa 1-50 mg/kg Körpergewicht vorzuziehen, während bei intravenöser Verabreichung eine Dosierung von etwa 0,1-10 mg/kg Körpergewicht zweckmäßig ist.

Die Arzneimittel der vorliegenden Erfindung werden in an sich bekannten Lösungs-, Misch-, Granulier- oder Dragierverfahren hergestellt.

Für die orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesia, Magnesiumcarbonat, Kaliumphosphat, Milchzucker, Glucose, Magnesiumstearylfumarat oder Stärke, insbesondere Maisstärke verwendet werden. Dabei kann die Zubereitung sowohl als Trocken- und Feuchtgranulat erfolgen. Als ölige Trägerstoffe oder Lösungsmittel kommen beispielsweise pflanzliche oder tierische Öle in Betracht, wie Sonnenblumenöl und Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösungen, Suspensionen oder Emulsionen gebracht. Als Lösungsmittel kommen z.B. in Frage: Wasser, physiologische Kochsalzlösungen oder Alkohole, z.B. Ethanol, Propandiol oder Glycerin, daneben auch Zuckerlösungen wie Glucose-oder Mannitlösungen, oder auch eine Mischung aus den verschiedenen genannten Lösungsmitteln.

Prinzip des HIV-Protease-Tests

Als synthetisches Substrat ist ein Heptapeptid der Sequenz Ser-Phe-Asn-Phe-Pro-Gln-Ile geeignet. Diese Sequenz tritt im natürlichen Substrat der Protease auf und liefert bei der autokatalytischen Freisetzung nach einer Phe-Pro-Spaltung den N-terminalen Beginn der Proteasesequenz (Pro-Gln-Ile...).

Es wird eine klonierte HIV-Protease (Max-Planck-Institut für Molekulare Genetik, Berlin) benutzt.

$$\text{Ser-Phe-Asn-Phe-Pro-Gln-Ile} \xrightarrow{\text{Protease}} \text{Ser-Phe-Asn-Phe} + \text{Pro-Gln-Ile}$$

Von den Reaktionsprodukten wird Ser-Phe-Asn-Phe zur Abtrennung und quantitativen Bestimmung mittels HPLC herangezogen.

Durchführung

| | | |
|---|---|---|
| Testansatz: | Substratlösung | 10 µl |
| | Inhibitorlösung bzw. MGTE 15-Puffer/DMSO (2,5 %) | 10 µl |
| | Proteaselösung | 5 µl |
| | | 23 µl |

Nach 2 Stunden bei 37 °C werden die Ansätze mit 225 µl HPLC-Elutionsmittel (s.u.) gestoppt. Etwa 150 µl des zentrifugierten Überstands (Eppendorf-Zentrifuge) werden in Mikrovials gefüllt. Das Injektionsvolumen für die HPLC-Trennung beträgt 20 µl. Ser-Phe-Asn-Phe wird durch Vergleich mit einem externen Standard bekannter Konzentration (0,5 µg/ 20 µl) quantitativ bestimmt und stellt die Meßgröße der Enzymaktivität (µg Tetrapeptid pro 20 µl Meßprobe) dar. Bezugswert (= 100 % Enzymaktivität) ist der Mittelwert aus 2 oder mehr Ansätzen ohne Inhibitor. Als Standard-Inhibitor wird Pepstatin A ($10^{-4}$ oder $10^{-5}$ M) verwendet.

Erforderliche Lösungen

a) Substratlösung: 2 mg Ser-Phe-Asn-Phe-Pro-Gln-Ile werden in 1 ml MGTE 15-Puffer gelöst.

b) Testsubstanzen: Diese werden $10^{-2}$ oder $10^{-3}$ M in Dimethylsulfoxid (DMSO) gelöst und 1 : 40 mit MGTE 15-Puffer verdünnt; 10 µl dieser Lösung werden pro Ansatz verwendet; die Endkonzentration des Präparates beträgt $10^{-4}$ bzw. $10^{-5}$ M; die DMSO-Konzentration in allen Testansätzen ist 1 %. Für $IC_{50}$-Bestimmungen wird das Präparat, ausgehend von $10^{-2}$ M, mit 100 % DMSO in der geeigneten Weise verdünnt.

c) Proteaselösung: Das Enzym wird mit MGTE 25-Puffer auf eine geeignete Konzentration verdünnt.

d) MGTE 15/25-Puffer

20 mM Morpholinoethansulfonsäure (MES)
15 (25) % (w/v) Glyzerin
0,1 % (v/v) Triton® X 100 (vgl. Merck Index 10, 6601)
5 mM EDTA
0,5 M NaCl

1 mM Dithiothreit (nur für MGTE 25)
1 mM Phenylmethylsulfonylfluorid (PMSF)
Der pH-Wert soll ca. 6,0 betragen.

e) HPLC-Elutionsmittel:

Eine 0,1 M Phosphorsäure-Lösung wird mit Triethylamin auf pH 2,5 eingestellt. Aus dieser Lösung wird ein Phosphorsäure-Acetonitril-Gemisch mit 22,6 Gewichts-Prozent Acetonitril hergestellt.

Bedingungen für die HPLC-Analyse:

Säule: Radial Pak LC Cartridge, 8 mm/C18, 5 µ (der Fa. Waters, USA).
Fluß: 1 ml/min
Detektoreinstellung: 215 nm, 0,08 AU
Zeit pro Analyse: 12 min; Retentionszeiten: Heptapeptid ca. 9,5 min, Tetrapeptid ca. 5,2 min
Laufmittel: 77.4 Gew.-% 0,1 M $H_3PO_4$/pH 2,5
22,6 Gew.-% Acetonitril

Die Testergebnisse, die die Wirksamkeit der erfindungsgemäßen Verbindungen belegen, sind in der nachfolgenden Tabelle aufgeführt.

Tabelle

| Beispiel Nr. | Restakt. bei $10^{-4}$ M | $IC_{50}$ [M] |
|---|---|---|
| 1 | 28 % | $4 \cdot 10^{-5}$ |
| 2 | 74 % | |
| 3 | 61 % | |
| 4 | 96 % | |
| 5 | 38 % | $7 \cdot 10^{-5}$ |
| 6 | 41 % | |
| 9 | 93 % | |
| 10 | 77 % | |
| 11 | 96 % | |
| 12 | 64 % | |
| 13 | 35 % | |
| 14 | 75 % | |
| 15 | 86 % | |
| 16 | 70 % | |
| 17 | 52 % | $2 \cdot 10^{-4}$ |
| 18 | 36 % | |
| 19 | 37 % | |
| 20 | 27 % | |

Fortsetzung Tabelle

| Beispiel Nr. | Restakt. bei $10^{-4}$ M | $IC_{50}$ [M] |
|---|---|---|
| 24 | 78 % | |
| 27 | 77 % | |
| 28 | 7 % | $7 \cdot 10^{-6}$ |
| 30 | 52 % | |
| 31 | 59 % | |
| 32 | 25 % | $4 \cdot 10^{-5}$ |
| 33 | 61 % | |
| 35 | 22 % | $4 \cdot 10^{-5}$ |
| 38 | 11 % | $9 \cdot 10^{-6}$ |
| 40 | 46 % | |
| 41 | 35 % | |
| 44 | 72 % | |
| 45 | 49 % | $1 \cdot 10^{-4}$ |
| 46 | 45 % | $4 \cdot 10^{-5}$ |
| 47 | 16 % | $1 \cdot 10^{-5}$ |
| 48 | 32 % | $6 \cdot 10^{-5}$ |
| 49 | 46 % | |
| 50 | 53 % | |
| 51 | 5 % | $5 \cdot 10^{-6}$ |
| 52 | 49 % | $1 \cdot 10^{-4}$ |
| 53 | 10 % | $1 \cdot 10^{-5}$ |

Verzeichnis der verwendeten Abkürzungen:

Ac Acetyl
Boc tert.-Butoxycarbonyl
BuLi n-Butyllithium
DC Dünnschichtchromatographie
DCC Dicyclohexylcarbodiimid
DCI Desorption Chemical Ionisation
DIP Diisopropylether
DNP 2,4-Dinitrophenyl
DMF Dimethylformamid
DMSO Dimethylsulfoxid
EE Essigsäureethylester
EI Electron Impact
Etoc Ethoxycarbonyl
FAB Fast atom bombardment
H Hexan

HOBt 1-Hydroxybenzotriazol
Iva Isovaleryl
M Molekularpeak
MeOH Methanol
MS Massenspektrum
MTB Methyl-tert.-butylether
Nva Norvalin
Nle Norleucin
R.T. Raumtemperatur
Schmp. Schmelzpunkt
$Sdp_{xx}$ Siedepunkt bei xx Torr
Thi ß-2-Thienylalanin
THF Tetrahydrofuran
Z Benzyloxycarbonyl.

Die sonstigen für Aminosäuren verwendeten Abkürzungen entsprechen dem in der Peptidchemie üblichem drei Buchstaben-Code wie er z.B. in Europ. J. Biochem. 138, 9 - 37 (1984) beschrieben ist. Falls nicht ausdrücklich anders angegeben, handelt es sich immer um Aminosäuren der L-Konfiguration.

Die nachstehenden Beispiele dienen zur Erläuterung der vorliegenden Erfindung, ohne daß diese darauf beschränkt wäre.

## Beispiel 1

Iva-Phe-Nva-[Cyclohexylmethyl,(4,4,5,5-tetramethyl-1,3,2-dioxaborolan-2-yl)]methylamid

139 mg Iva-Phe-Nva-OH werden in 5 ml THF gelöst und bei -20° C erst 44 µl N-Methylmorpholin, dann 52 µl Chlorameisensäureisobutylester zugespritzt. Nach 5 Minuten bei -20° C wird 56 µl Triethylamin in 2 ml THF addiert. Das so erhaltene gemischte Anhydrid wird bei -20° C zu einer Lösung von 2- (1′-Amino-2′-cyclohexyl)ethyl],4,4,5,5-tetramethyl-1,3,2-dioxaborolan (Trifluoressigsäuresalz) in 5 ml THF gespritzt, bei -20° C 1 h, bei R.T. weitere 2 h gerührt, THF im Vakuum entfernt und in 20 ml Essigester aufgenommen. 2 mal wird mit 20 ml gesättigter wäßriger $NaHCO_3$, 2 mal mit 20 ml 0,06 M $KH_2PO_4$ extrahiert. Anschließend wird über $Na_2SO_4$ getrocknet, das Solvens im Vakuum entfernt und an Kieselgel mit MTB chromatographiert. Man erhält 105 mg der Titelverbindung als farbloses amorphes Pulver.
$R_f$(MTB) = 0.38
MS(FAB): 584 (M + 1)

a) 2-[(1′-Amino-2′-cyclohexyl)ethyl],4,4,5,5-tetramethyl-1,3,2-dioxaborolan (Trifluoressigsäuresalz)

0,7 ml Hexamethyldisilazan werden in 8 ml THF gelöst und bei -78° C mit 2,1 ml 1,6 M BuLi in Hexan versetzt. Man läßt 5 Minuten bei R.T. rühren, kühlt erneut auf -78° C ab und gibt 1,1 g 2-[(1′-Jod-2′-cyclohexyl)ethyl],4,4,5,5-tetramethyl-1,3,2-dioxaborolan in 5 ml THF zu. 22 h wird bei R.T. gerührt, dann das THF im Vakuum entfernt und der Rückstand in 4 ml Diethylether aufgenommen. Anschließend wird bei 0° C 0,3 ml Trifluoressigsäure langsam zugetropft und 10 Minuten bei dieser Temperatur nachgerührt, wobei ein weißer kristalliner Niederschlag ausfällt. Dieser wird unter Argonatmosphäre abfiltriert und mit auf 0° C gekühltem Diethylether gewaschen. Man erhält 650 mg der Titelverbindung als weiße Kristalle.
Schmp. 154-157° C
MS(DCI) : 254 (M + 1)

b) 2-[(1′-Jod-2′-cyclohexyl)ethyl],4,4,5,5-tetramethyl-1,3,2-dioxaborolan

3,6 g NaI werden in 50 ml Acetonitril gelöst und 4,3 g 2-[(1′-Chlor-2′-cyclohexyl)ethyl],4,4,5,5-tetramethyl-1,3,2-dioxaborolan gelöst in 20 ml Acetonitril bei R.T. zugetropft. Nach etwa 5 Minuten beginnt NaCl auszufallen. Nach 3 stündigem Rühren bei R.T. wird die Lösung abfiltriert, das Acetonitril im Vakuum entfernt und an Kieselgel mit MTB/H 1:10 chromatographiert. Man erhält 4,4 g der Titelverbindung als blaßgelbes Öl.

$R_f$ (EE/H 1:8) = 0,59
MS (EI) : 364 (M)

c) 2-[(1′-Chlor-2′-cyclohexyl)ethyl],4,4,5,5-tetramethyl-1,3,2-dioxaborolan

6,8 g $CH_2Cl_2$ werden in 80 ml THF gelöst und auf -100 °C abgekühlt. Nun wird 31,3 ml 1,6 M BuLi auf -78 °C vorgekühlt und so langsam zugetropft, daß die Temperatur im Reaktionskolben nicht über -95 °C ansteigt. Anschließend läßt man 11,2 g auf -78 °C vorgekühltes 2-Cyclohexylmethyl,4,4,5,5-tetramethyl-1,3,2-dioxaborolan in 25 ml Diethylether zulaufen. Anschließend wird 20 h bei R.T. gerührt, die Lösung in 300 ml Phosphatpuffer (pH = 7) eingerührt und 3 mal mit je 200 ml MTB extrahiert, über $Na_2SO_4$ getrocknet, das Solvens im Vakuum entfernt und an Kieselgel mit EE/H 1:8 chromatographiert. Man erhält 5,3 g der Titelverbindung als farbloses Öl.
$R_f$ (EE/H 1:8) = 0,50
MS (EI) : 272 (M)

d) 2-Cyclohexylmethyl,4,4,5,5-tetramethyl-1,3,2-dioxaborolan

Zu 12,0 g Magnesium und 200 ml Diethylether wird 62,9 ml Cyclohexylmethylbromid langsam zugetropft. Nach Auflösung des Magnesiums wird die Lösung der Grignard-Verbindung zu einer auf -78 °C vorgekühlten Lösung von 50,3 ml Trimethylborat in 100 ml Diethylether so zugetropft, daß die Temperatur nicht über -55 °C ansteigt und anschließend bei R.T. 2 h gerührt. Dann wird unter Eiskühlung 113 ml 40 %ige $H_2SO_4$ so zugegeben, daß die Innentemperatur nicht über 25 °C ansteigt. Nun wird 53,2 g Pinakol in 100 ml Diethylether zugegossen und an einem Rotationsverdampfer der Diethylether sowie das Nebenprodukt Methanol entfernt. Anschließend wird mit $NaHCO_3$ auf pH = 8 eingestellt, 3 mal mit 200 ml MTB extrahiert, über $Na_2SO_4$ getrocknet, das Solvens im Vakuum entfernt und im Feinvakuum destilliert.

Man erhält 49,7 g der Titelverbindung als farbloses Öl.
$Sdp_{0.05}$ = 57 °C
MS (EI) : 224 (M)

Die Verbindung des Beispiels 2 wird analog Beispiel 1 hergestellt.

**Beispiel 2**

Iva-Phe-Nle-[Cyclohexylmethyl,(4,4,5,5-tetramethyl-1,3,2-dioxaborolan-2-yl)]methylamid

$R_f$ (MTB) = 0,46
MS (FAB) : 598 (M + 1)

**Beispiel 3**

Iva-Phe-His-[Cyclohexylmethyl,(4,4,5,5-tetramethyl-1,3,2-dioxaborolan-2-yl)]methylamid

230 mg Iva-Phe-His(DNP)-[Cyclohexylmethyl,(4,4,5,5-tetramethyl-1,3,2-dioxaborolan-2-yl)]methylamid werden in 5 ml Acetonitril gelöst und 39 ml Thiophenol addiert. 2 h wird bei R.T. gerührt, anschließend das Solvens im Vakuum entfernt und an Kieselgel mit Aceton chromatographiert. Man erhält 65 mg der Titelverbindung als farbloses Harz.
$R_f$ (Aceton) = 0,58
MS (FAB) : 622 (M + 1)

a) Iva-Phe-His(DNP)-[Cyclohexylmethyl,(4,4,5,5-tetramethyl-1,3,2-dioxaborolan-2-yl)]methylamid wird analog Beispiel 1 hergestellt.

Die Verbindung des Beispiels 4 wird analog Beispiel 3 hergestellt.

**Beispiel 4**

Iva-Thi-His-[Cyclohexylmethyl,(4,4,5,5-tetramethyl-1,3,2-dioxaborolan-2-yl)methylamid

$R_f$ (MTB) = 0.48
MS (FAB) : 62B (M + 1)

**Beispiel 5**

Iva-Phe-Nva-(Cyclohexylmethyl,dihydroxyboryl)methylamid

0,86 ml einer 0,5 M Lösung von $BCl_3$ in $CH_2Cl_2$ werden auf -78 °C abgekühlt und 100 mg der Titelverbindung aus Beispiel 1 in 0,86 ml $CH_2Cl_2$ langsam zugetropft. 1 h wird anschließend bei 0 °C gerührt, in 10 ml 0,1 N NaOH gegossen und 2 mal mit 10 ml EE gewaschen. Dann wird die wäßrige Phase mit HCl auf etwa pH = 2 angesäuert, 3 mal mit 10 ml EE extrahiert, über $Na_2SO_4$ getrocknet, das Solvens im Vakuum entfernt und an Kieselgel mit Aceton/Wasser 10 : 1 chromatographiert. Man erhält 20 mg der Titelverbindung als farblosen Schaum.
$R_f$ (Aceton $H_2O$ 10:1) = 0,45 - 0,60
MS (FAB, Glycerinmatrix):
557 (M + 56)

**Beispiel 6**

Iva-Phe-Nva-[Cyclohexylmethyl,[(N-B)-(2,2′-iminodiethanolato)-boryl]]methylamid

74 mg der Titelverbindung des Beispiels 1 werden in 5 ml EE suspendiert. Anschließend werden 12,2 µl Diethanolamin addiert und bei R.T. im Ultraschallbad 5 h gerührt. Das Solvens wird im Vakuum entfernt und der $CH_2Cl_2$-lösliche Teil des Reaktionsgemisches an Kieselgel mit MTB/H 5:1 chromatographiert. Man erhält 7 mg der Titelverbindung als amorphes Pulver.
$R_f$ (MTB) = 0,37

Anschließend wird die Säule mit Aceton/Wasser 10:1 eluiert und man erhält 41 mg der Titelverbindung des Beispiels 5.

Die Verbindung des Beispiels 7 wird analog Beispiel 1 hergestellt.

**Beispiel 7**

Iva-Phe-Thi-[Cyclohexylmethyl,(4,4,5,5-tetramethyl-1,3,2-dioxaborolan-2-yl)]methylamid

$R_f$ (MTB) = 0,50
MS (FAB): 638 (M + 1)

**Beispiel 8**

Iva-Phe-Thi-(Cyclohexylmethyl,dihydroxyboryl)methylamid

165 mg der Titelverbindung aus Beispiel 7 werden in 5 ml EE gelöst und 25 µl Diethanolamin zugegeben. 5 h wird bei R.T. im Ultraschallbad gerührt, wobei der Diethanolaminester der Titelverbindung ausfällt. Das Solvens wird im Vakuum entfernt und an Kieselgel mit Aceton/$H_2O$ = 10:1 chromatographiert. Man erhält 62 mg der Titelverbindung als farblosen Schaum.

$R_f$ (Aceton/$H_2O$ 10:1) = 0.45 - 0.55
MS (FAB, Glycerinmatrix} : 611 (M+56)

Die Verbindung des Beispiels 9 wird analog Beispiel 8 aus der Titelverbindung des Beispiels 4 hergestellt.

**Beispiel 9**

Iva-Thi-His-(Cyclohexylmethyl,dihydroxyboryl)methylamid

$R_f$ (Aceton/$H_2O$ 10:1) = 0.10
MS(FAB, Glycerinmatrix) : 601 (M+56)

Die Verbindungen der Beispiele 10 und 11 werden analog Beispiel 1 hergestellt.

**Beispiel 10**

Iva-Phe-Nva-[(1-Naphthylmethyl),(4,4,5,5-tetramethyl-1,3,2-dioxaborolan-2-yl)]methylamid

$R_f$ (MTB) = 0.45
MS (FAB): 628 (M+1)

**Beispiel 11**

Iva-Phe-Nva- Cyclooctylmethyl,(4,4,5,5-tetramethyl-1,3,2-dioxaborolan-2-yl)]methylamid

$R_f$ (MTB) = 0.43
MS (FAB): 612 (M+1)

Die Verbindungen der Beispiele 12-16 werden analog Beispiel 1 hergestellt:

**Beispiel 12**

Iva-Phe-Nva-[Cyclohexylmethyl, (-)-Pinandioxyboryl]methylamid

$R_f$ (EE/H 1:1) = 0,19
MS (FAB): 636 (M+1)

**Beispiel 13**

Iva-Phe-Nva-[Cycloundecylmethyl, (4,4,5,5-tetramethyl-1,3,2-dioxaborolan-2-yl)]methylamid

$R_f$ (MTB) = 0,50
MS (FAB): 550 (M+1)

**Beispiel 14**

Iva-Phe-Nva-[(4-Methylcyclohexyl)methyl,(4,4,5,5-tetramethyl-1,3,2-dioxaborolan-2-yl)]methylamid

$R_f$ (EE/H 1:1) = 0,10
MS (FAB): 598 (M+1)

**Beispiel 15**

Iva-Phe-Nva- (Cycloheptylmethyl,(4,4,5,5-tetramethyl-1,3 2-dioxaborolan-2-yl)]methylamid

$R_f$ (MTB) = 0,35
MS (FAB): 598 (M+1)

**Beispiel 16**

Iva-Phe-(N-Me-Phe)-cyclohexylmethyl,(4,4,5,5-tetramethyl-1,3,2-dioxaborolan-2-yl)]methylamid

$R_f$ (DIP MTB 1:1) = 0,20
MS (FAB): 646 (M+1)

Die Verbindungen der Beispiele 17-19 werden analog Beispiel 3 hergestellt:

**Beispiel 17**

Iva-Phe-His-[Cyclooctylmethyl,(4,4,5,5-tetramethyl-1,3,2-dioxaborolan-2-yl)]methylamid

$R_f$ (Aceton) = 0,10
MS (FAB): 650 (M+1)

**Beispiel 18**

Iva-Phe-His-[Cycloheptylmethyl,(4,4,5,5-tetramethyl-1,3,2-dioxaborolan-2-yl)]methylamid

$R_f$ (Aceton) = 0,12
MS (FAB): 634 (M+1)

**Beispiel 19**

Bis-(1-Naphthylmethyl)acetyl-His-[3-methyl,l-(4,4,5,5-tetramethyl-1,3,2-dioxaborolan-2-yl)]butylamid

$R_f$ (Aceton) = 0,20
MS (FAB): 673 (M+1)

Die Verbindungen der Beispiele 20-33 werden aus den entsprechenden 1,3,2-Dioxaborolanen analog Beispiel 8 hergestellt.

**Beispiel 20**

Iva-Phe-Nva-(Cyclooctylmethyl,dihydroxyboryl)methylamid

$R_f$ (Aceton/$H_2O$ 10:1) = 0,54
MS (FAB, Glycerin): 586 (M+57).

**Beispiel 21**

Iva-Phe-Nva-(Cycloheptylmethyl,dihydroxyboryl)methylamid

$R_f$ (Aceton/$H_2O$ 10:1) = 0.47
MS (FAB. Glycerin): 572 (M+57)

**Beispiel 22**

Iva-phe-Nva-(4-methylcyclohexyl)methyl,dihydroxyboryl]methylamid

$R_f$ (Aceton $H_2O$ 10:1) = 0,57
MS (FAB, Glycerin): 572 (M+57)

**Beispiel 23**

Iva-Phe-His-(Cycloheptylmethyl,dihydroxyboryl)methylamid

$R_f$ (Aceton $H_2O$ 10:1) = 0,14
MS (FAB, Glycerin): 610 (M+57)

**Beispiel 24**

Iva-Phe-His-(Cyclooctylmethyl,dihydroxyboryl)methylamid

$R_f$ (Aceton/$H_2O$ 5:1) = 0,29
MS (FAB, (Glycerin): 624 (M+57)

**Beispiel 25**

Iva-Phe-Nva-(Cycloundecylmethyl,dihydroxyboryl)methylamid

$R_f$ (Aceton $H_2O$ 15:1) = 0,45
MS (FAB, Glycerin): 628 (M+57)

**Beispiel 26**

(3-t-Butylsulfonyl)propionyl-Nva-(Cycloundecylmethyl, dihydroxyboryl)methylamid

$R_f$ (Aceton/$H_2O$ 10:1) = 0,57
MS (FAB, Glycerin): 573 (M+57)

**Beispiel 27**

(3-t-Butylsulfonyl)propionyl-Nva-(1-Naphthylmethyl, dihydroxyboryl)methylamid

$R_f$ (Aceton/$H_2O$ 10:1) = 0,29
MS (FAB, Glycerin): 547 (M+57)

**Beispiel 28**

(2-Benzyl,3-t-butylsulfonyl)propionyl-Val-(Cyclohexylmethyl,dihydroxyboryl)methylamid

$R_f$ (Aceton/$H_2O$ 10:1) = 0,57
MS (FAB, Glycerin): 593 (M+57)

**Beispiel 29**

[3-t-Butylsulfonyl,2-(2-thienylmethyl)]propionyl-Nva-(Cycloheptylmethyl,dihydroxyboryl)methylamid

$R_f$ (Aceton/$H_2O$ 10:1) = 0,55
MS (FAB, Glycerin): 613 (M+57)

**Beispiel 30**

[3-t-Butylsulfonyl,2-(2-thienylmethyl)]propionyl-Nva-(Cyclohexylmethyl,dihydroxyboryl)methylamid

$R_f$ (Aceton/$H_2O$ 10:1) = 0,44
MS (FAB, Glycerin): 599 (M+57)

**Beispiel 31**

[3-t-Butylsulfonyl,2,(2-thienylmethyl)]propionyl-Thi-(Cyclohexylmethyl,dihydroxyboryl)methylamid

$R_f$ (Aceton/$H_2O$ 20:1) = 0,35
MS (FAB, Glycerin) 653 (M+57)

**Beispiel 32**

(2-Benzyl,3-t-butylsulfonyl)propionyl-Nva-(Cyclopentylmethyl,dihydroxyboryl)methylamid

$R_f$ (Aceton/$H_2O$ 10:1) = 0,41
MS (FAB, Glycerin): 579 (M+57)

**Beispiel 33**

[3-t-Butylsulfonyl,2-(2-thienylmethyl)]propionyl-Thi-(Cycloheptylmethyl,dihydroxyboryl)methylamid

$R_f$ (Aceton/$H_2O$ 10:1) = 0,60
MS (FAB, Glycerin): 667 (M+57)

**Beispiel 34**

[3-t-Butylsulfonyl,2-(2-thienylmethyl)]propionyl-Nva-[Cyclohexylmethyl,(4,4,5,5-tetramethyl-1,3,2-dioxaborolan-2-yl)]methylamid

Die Titelverbindung wird analog Beispiel 1 aus [3-t-Butylsulfonyl,2-(2-thienylmethyl)]propionyl-Nva-OH

und der Titelverbindung des Beispiels 1a hergestellt.
$R_f$ (MTB) = 0,40
MS (FAB): 625 (M + 1)

a) [3-t-Butylsulfonyl,2-(2-thienylmethyl)]propionyl-Nva-OH

490 mg [3-t-Butylsulfonyl,2-(2-thienyl)]propionyl-Nva-OMe werden in 4 ml Methanol gelöst, 0,4 ml $H_2O$ sowie 0,7 ml 2N NaOH zugegeben und 5h bei R.T. gerührt. Mit $NaHSO_4$-Lösung wird auf pH = 2 angesäuert und 3 x mit 50 ml EE extrahiert. Anschließend wird über $Na_2SO_4$ getrocknet und das Solvens im Vakuum entfernt. Man erhält 470 mg blaßgelbes Harz.
$R_f$ (EE) = 0,24
MS (FAB): 390 (M + 1)

b) [3-t-Butylsulfonyl,2-(2-thienylmethyl)]propionyl-Nva-OMe

1.5 g [3-t-Butylsulfonyl,2-(2-thienylmethyl)]propionsäure sowie 0,95 g Nva-OMe x HCl werden in 40 ml $CH_2Cl_2$ gelöst und bei 10 °C zunächst 3,8 ml Triethylamin, dann 3,5 ml einer 50 % Lösung von Propanphosphonsäure-Anhydrid in $CH_2Cl_2$ zugegeben. 20 h wird bei R.T. gerührt, das Solvens im Vakuum entfernt, in 100 ml MTB aufgenommen und mit je 100 ml $NaHSO_4$-Lösung und $NaHCO_3$-Lösung gewaschen. Über $Na_2SO_4$ wird getrocknet, das Solvens im Vakuum entfernt und mit EE/H 1:1 chromatographiert. Man erhält 2 diastereomere Öle, die getrennt weiter verarbeitet werden.

| | | |
|---|---|---|
| Diastereomer 1 | $R_f$ (EE/H 1:1) = 0,30 | 0,95 g |
| Diastereomer 2 | $R_f$ (EE/H 1:1) = 0,20 | 0,95 g |

MS (DCI, für beide Diastereomere gleich): 404 (M + 1)

c) [3-t-Butylsulfonyl,2-(2-thienylmethyl)]propionsäure

4,4 g [3-t-Butylsulfonyl,2-(2-thienylmethyl)]propionsäuremethylester werden in 50 ml 5N HCl suspendiert und 2 h zum Rückfluß erhitzt. Nach Abkühlung wird 3 x mit 50 ml EE extrahiert, über $Na_2SO_4$ getrocknet und das Solvens im Vakuum entfernt. Man erhält 4,0 g der Titelverbindung als blaßgelbes Öl.
$R_f$ (MTB) = 0,15 - 0,25
MS (DCI): 291 (M + 1)

d) [3-t-Butylsulfonyl,2-(2-thienylmethyl)]propionsäuremethylester

8,4 g [3-t-Butylthio,2-(2-thienylmethyl)]propionsäuremethylester werden in 100 ml $CH_2Cl_2$ gelöst und 10,6 g m-Chlorperbenzoesäure unter Eiskühlung portionsweise zugegeben. 1 h wird bei R.T. gerührt, anschließend zunächst mit 100 ml 10 % $Na_2SO_3$, dann mit 100 ml $NaHCO_3$ gewaschen. Über $Na_2SO_4$ wird getrocknet und das Solvens im Vakuum entfernt. Man erhält 7,2 g der Titelverbindung als farbloses Öl.
$R_f$ (MTB) = 0,56
MS (DCI): 305 (M + 1)

e) [3-t-Butylthio,2-(2-thienylmethyl)]propionsäuremethylester

6,1 ml t-Butylmercaptan werden in 100 ml MeOH (wasserfrei) gelöst und unter Argon 130 mg NaH zugegeben. Anschließend werden 7,6 g 2-(2-Thienylmethyl)acrylsäuremethylester zugetropft und 4 h bei R.T. gerührt. Das Solvens wird im Vakuum entfernt, in 100 ml MTB aufgenommen und mit 100 ml 5 % $NaHSO_4$-Lösung gewaschen. Über $Na_2SO_4$ wird getrocknet und das Solvens im Vakuum entfernt. Man erhält 10,4 g der Titelverbindung als blaßgelbe Flüssigkeit, die ohne Reinigung und Charakterisierung weiter

eingesetzt wird.
$R_f$ (DIP/H 1:5) = 0,31

f) 2-(2-Thienylmethyl)acrylsäure-methylester

11,8 g 2-Thienylmethylmalonsäure-monomethylester, 5,8 ml Diethylamin und 5,0 ml 36 % wäßrige Formaldehyd-Lösung werden bei R.T. unter Argon 1 h gerührt. Das Wasser wird anschließend im Vakuum entfernt und der Rückstand chromatographiert. Man erhält 7,6 g der Titelverbindung als farblose Flüssigkeit.
$R_f$ (MTB/H 1:5) = 0,49

g) 2-Thienylmethylmalonsäure-monomethylester

20,1 g 2-Thienylmethylmalonsäure-dimethylester werden in 300 ml MeOH gelöst und 5,0 g KOH zugegeben. Bei R.T. wird 7 h gerührt, das Wasser im Vakuum entfernt und mit je 100 ml 5 % $Na_2CO_3$-Lösung und EE aufgenommen. Anschließend wird die wäßrige Phase auf pH = 2 angesäuert und 3 x mit je 100 ml EE extrahiert. Über $Na_2SO_4$ wird getrocknet und das Solvens im Vakuum entfernt. Man erhält 16,0 g der Titelverbindung als blaßgelbes Öl.
$R_f$ (EE MeOH 6:1) = 0,3 - 0,4

h) 2-Thienylmethylmalonsäure-dimethylester

87,8 g Malonsäuredimethylester und 41,0 g Kalium-t-butylat werden unter Eiskühlung in 1,1 1 THF (wasserfrei) gelöst und unter Argon 44,1 g 2-Thienylmethylchlorid in 500 ml THF zugetropft. 3 h wird bei R.T. gerührt, das KCl abfiltriert, das Solvens im Vakuum entfernt und der Rückstand chromatographiert. Man erhält 33,8 g der Titelverbindung (I) als farbloses Öl neben 8,8 g Bis-(2-Thienylmethyl)-malonsäuredimethylester (II)
$R_f$ (I) (Toluol/DIP 20:1) = 0,35
$R_f$ (II) (Toluol/DIP 20:1) = 0,44

g) 2-Thienylmethylchlorid

252 g Thiophen werden in 128 ml konzentrierter wäßriger HCl suspendiert und bei 0 °C 1 h lang HCl-Gas eingeleitet. Anschließend werden ohne Unterbrechung des HCl-Stromes 255 ml 35 % wäßrige Formaldehydlösung zugetropft und weitere 15 Minuten bei 0 °C gerührt. Nach Abtrennung der organischen Phase wird die wäßrige Phase noch 2 x mit 600 ml $CH_2Cl_2$ extrahiert. Anschließend wird 2 x mit 600 ml gesättigter wäßriger $Na_2CO_3$ gewaschen, über $Na_2SO_4$ getrocknet das Solvens im Vakuum entfernt und destilliert. Man erhält 174 g der Titelverbindung als farblose Flüssigkeit.
$Sdp_{22}$ = 81 - 84 °C

Die Verbindungen der Beispiele 35-55 werden analog Beispiel 34 hergestellt.

**Beispiel 35**

(2-Benzyl,3-t-butylsulfonyl)propionyl-Nva-[Cyclohexylmethyl,(4,4,5,5-tetramethyl-1,3,2-dioxaborolan-2-yl)-]methylamid

$R_f$ (MTB) = 0,38
MS (FAB): 619 (M + 1)

**Beispiel 36**

(3-t-Butylsulfonyl)propionyl-Nva-[Cycloundecylmethyl, (4,4,5,5-tetramethyl-1,3,2-dioxaborolan-2-yl)-]methylamid

$R_f$ (EE) = 0,24
MS (FAB): 599 (M + 1)

**Beispiel 37**

(3-t-Butylsulfonyl)propionyl-Nva-[1-Naphthylmethyl, (4,4,5,5-tetramethyl-1,3,2-dioxaborolan-2-yl)]methylamid

$R_f$ (EE) = 0,23
MS (FAB): 573 (M + 1)

**Beispiel 38**

(2-Benzyl,3-t-butylsulfonyl)propionyl-Val-[Cyclohexylmethyl,(4,4,5,5-tetramethyl-1,3,2-dioxaborolan-2-yl)-]methylamid

$R_f$ (MTB) = 0,25
MS (FAB): 619 (M + 1)

**Beispiel 39**

[3-t-Butylsulfonyl,2-(2-thienylmethyl)]propionyl-Thi-[Cycloheptylmethyl,(4,4,5,5-tetramethyl-1,3,2-dioxaborolan-2-yl)]methylamid

$R_f$ (EE.H 1:1) = 0,23
MS (FAB): 693 (M + 1)

**Beispiel 40**

[3-t-Butylsulfonyl,2-(2-thienylmethyl)]propionyl-Nva-[Cycloheptylmethyl,(4,4,5,5-tetramethyl-1,3,2-dioxaborolan-2-yl)]methylamid

$R_f$ (MTB) = 0,35
MS (FAB): 639 (M + 1)

**Beispiel 41**

[3-t-Butylsulfonyl,2-(1-naphthylmethyl)]propionyl-Nva-[3-methyl,1-(4,4,5,5-tetramethyl-1,3,2-dioxaborolan-2-yl)]butylamid

$R_f$ (MTB) = 0,26
MS (FAB): 629 (M + 1)

**Beispiel 42**

[3-t-Butylsulfonyl,2-(2-thienylmethyl)]propionyl-Thi-[Cyclohexymethyl,(4,4,5,5-tetramethyl-1,3,2-dioxaborolan-2-yl)]methylamid

$R_f$ (MTB) = 0,53
MS (FAB): 679 (M+1)

**Beispiel 43**

(2-Benzyl,3-t-butylsulfonyl)propionyl-Nva-[Cyclopentylmethyl,(4,4,5,5-tetramethyl-1,3,2-dioxaborolan-2-yl)-]methylamid

$R_f$ (MTB) = 0,36
MS (FAB): 605 (M+1)

**Beispiel 44**

(2-Benzyl,3-t-butylsulfonyl)propionyl-Nva- Benzyl,(4,4,5,5-tetramethyl-1,3,2-dioxaborolan-2-yl)]methylamid

$R_f$ (MTB) = 0,30
MS (FAB): 613 (M+1)

**Beispiel 45**

(2-Benzyl,3-t-butylsulfonyl)propionyl-Asn-[Benzyl,(4,4,5,5-tetramethyl-1,3,2-dioxaborolàn-2-yl)]methylamid

$R_f$ (EE) = 0,05
MS (FAB): 628 (M+1)

**Beispiel 46**

(2-Benzyl,3-t-butylsulfonyl)propionyl-Nva- Cycloheptyl, (4,4,5,5-tetramethyl-1,3,2-dioxaborolan-2-yl)-]methylamid

$R_f$ (MTB) = 0,30
MS (FAB): 619 (M+1)

**Beispiel 47**

[3-t-Butylsulfonyl,2-(1-naphthylmethyl)]propionyl-Nva-[Cyclopentylmethyl,(4,4,5,5-tetramethyl-1,3,2-dioxaborolan-2-yl)]methylamid

$R_f$ (MTB)= 0,40
MS (FAB): 655 (M+1)

**Beispiel 48**

[3-t-Butylsulfonyl,2-(1-naphthylmethyl)]propionyl-Nva-[3-Ethyl,1-(4,4,5,5-tetramethyl-1,3,2-dioxaborolan-2-yl)-]pentylamid

$R_f$ (MTB) = 0,50
MS (FAB): 657 (M+1)

**Beispiel 49**

(2-Benzyl,3-t-butylsulfonyl)propionyl-Nva-[3-Ethyl,1-(4,4,5,5-tetramethyl-1,3,2-dioxaborolan-2-yl)]pentylamid

$R_f$ (MTB) = 0,44
MS (FAB): 607 (M+1)

**Beispiel 50**

(2-Benzyl,3-t-butylsulfonyl)propionyl-Nva-[2-Methyl,1-(4,4,5,5-tetramethyl-1,3,2-dioxaborolan-2-yl)]butylamid

$R_f$ (MTB) = 0,35
MS (FAB): 579 (M+1)

**Beispiel 51**

[3-t-Butylsulfonyl, 2-(1-naphthylmethyl)]propionyl-Val-[cyclohexylmethyl,(4,4,5,5-tetramethyl-1,3,2-dioxaborolan-2-yl)]methylamid

$R_f$ (MTB) = 0.50
MS (FAB): 667 (M+1)

**Beispiel 52**

[3-t-Butylsulfonyl, 2-(2-thienylmethyl)]propionyl-Asn-[cyclohexylmethyl,(4,4,5,5-tetramethyl-1,3,2-dioxaborolan-2-yl)]-methylamid

$R_f$ (EE Aceton 5:1) = 0,30
MS (FAB): 640 (M+1)

**Beispiel 53**

[3-t-Butylsulfonyl,2-(1-naphthylmethyl)]propionyl-Asn-[cyclohexylmethyl,(4,4,5,5-tetramethyl-1,3,2-dioxaborolan-2-yl)]methylamid

$R_f$ (EE/Aceton 5:1) = 0,25
MS (FAB): 684 (M+1)

[3-t-Butylsulfonyl,2-(2-methyl)benzyl]propionyl-Nva-[Cyclohexylmethyl,(4,4,5,5-tetramethyl-1,3,2-dioxaborolan-2-yl)]methylamid

$R_f$ (MTB) = 0,40
MS (FAB): 633 (M + 1)

**Beispiel 54**

[3-t-Butylsulfonyl,2-(3-methyl)benzyl]propionyl-Nva-[Cyclohexylmethyl,(4,4,5,5-tetramethyl-1,3,2-dioxaborolan-2-yl)]methylamid

$R_f$ (MTB) = 0,40
MS (FAB): 633 (M + 1)

**Beispiel 55**

[3-t-Butylsulfonyl,2-(3-methyl)benzyl]propionyl-Nva-[Cyclohexylmethyl,(4,4,5,5-tetramethyl-1,3,2-dioxaborolan-2-yl)methylamid

$R_f$ (MTB) = 0,40
MS (FAB): 633 (M + 1)

**Beispiel 56**

Kapseln, die für die orale Verabreichung geeignet sind, enthalten die nachfolgend genannten Bestandteile und können auf an sich bekannte Weise hergestellt werden, indem man Wirk- und Hilfsstoffe vermischt und in Gelantinekapseln abfüllt. Diese Kapseln dienen zur antiviralen Behandlung in einer Dosis von einer Kapsel 2-4 mal täglich.

| Bestandteile (pro Kapsel) | Gewicht |
|---|---|
| Verbindung gemäß Beispiel 51 | 250 mg |
| Milchzucker | 200 mg |
| Talkum | 10 mg |
| kolloidales Siliziumdioxid | 10 mg |

**Bespiel 57**

Wirkstofflösungen, die zur Injektion geeignet sind, enthalten die nachfolgend genannten Bestandteile und können auf an sich bekannter Weise hergestellt werden, indem die Stoffe miteinander vermischt und in sterile Ampullen abgefüllt werden. Die Injektionslösungen dienen zur antiviralen Behandlung in einer Dosis von 1-2 Injektionseinheiten (1 Injektionseinheit = 1 Ampulle) pro Tag.

| Bestandteile (pro Ampulle) | Gewicht |
|---|---|
| Verbindung gemäß Beispiel 51 | 150 mg |
| Glucose | 150 mg |
| Natriumchlorid | 50 mg |
| Methylparaben | 5 mg |
| steriles Wasser | 5 g |

**Ansprüche**

1. Verwendung von Verbindung der Formel I

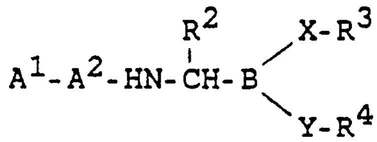 (I)

in welcher
A' einen Rest der Formeln II, III, IV, V oder VI bedeutet

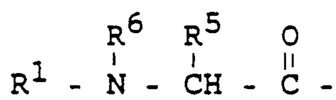 (II)

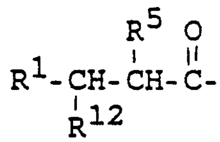 (III)

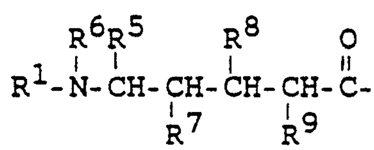 (IV)

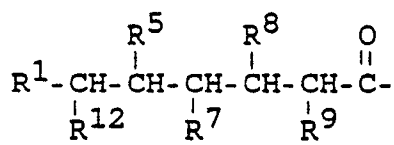 (V)

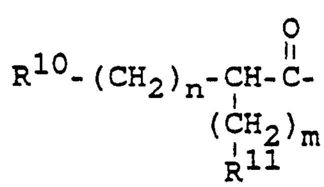 (VI)

worin
R' $a_1$) Wasserstoff, ($C_1$-$C_{12}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_8$)-Alkanoyloxy, Carboxy, ($C_1$-$C_7$)-Alkoxycarbonyl, F, Cl, Br, I, Amino, Carbamoyl, Amidino, das gegebenenfalls durch einen, zwei oder drei ($C_1$-$C_8$)-Alkylreste substituiert sein kann, Guanidino, das gegebenenfalls durch einen, zwei, drei oder vier ($C_1$-$C_8$)-Alkylreste substituiert sein kann, ($C_1$-$C_7$)-Alkylamino, Di-($C_1$-$C_7$)-alkylamino, ($C_1$-$C_5$)-Alkoxycarbonylamino, ($C_7$-$C_{15}$)-Aralkoxycarbonylamino und 9-Fluorenylmethoxycarbonylamino substituiert ist, mono-, bi- oder tricyclisches ($C_3$-$C_{18}$)-Cycloalkyl, ($C_3$-$C_{18}$)-Cycloalkyl-($C_1$-$C_6$)-alkyl oder ($C_6$-$C_{14}$)-Aryl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, gegebenenfalls mit bis zu 2 Halogenen substituiertes Anilino und Trifluormethyl substituiert ist, ($C_6$-$C_{14}$)-Aryl-($C_1$-$C_6$)-alkyl, worin der Arylteil gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylamino, Di-($C_1$-$C_7$)-

alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo- und Guanidino-($C_1$-$C_8$)-alkyl substituiert ist, oder für den Rest eines 5- oder 6-gliedrigen monocyclischen oder 9- oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht,und der gegebenenfalls wie ($C_6$-$C_{14}$)-Aryl unter $a_1$) definiert, mono-, di- oder trisubstituiert ist, bedeutet

oder

$a_2$) einen Rest der Formel VII bedeutet

$R^1$ - W , VIII

worin $R^1$ wie $R^1$ unter $a_1$) definiert ist und W für -CO-, -O-CO-, $-SO_2-$, -SO-, $-HN-SO_2-$, -HN-CO-, -CH(OH)- oder -N(OH)- steht,

$R^2$, $R^5$ und $R^9$ unabhängig voneinander wie $R^1$ unter $a_1$) definiert sind,

$R^3$ und $R^4$ unabhängig voneinander Wasserstoff oder ($C_1$-$C_{12}$)-Alkyl bedeuten, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, oder zusammen mit Bor, X und Y ein mono-, bi- oder tricyclisches, gesättigtes oder teilweise ungesättigtes mono-, di-, tri- oder tetra-($C_1$-$C_{12}$)alkyliertes Ringsystem mit 5-18 Ringgliedern, das außer Bor, X, Y und Kohlenstoff noch ein -O- Glied ein $-NR'^3$-Glied oder ein $-CR'^4R'^5$-Glied enthalten kann, bilden,

X und Y unabhängig voneinander für -O- oder $-NR'^3$-stehen,

$R^6$ für Wasserstoff oder ($C_1$-$C_8$)-Alkyl steht, oder zusammen mit $R^5$ ein mono- oder bicyclisches, gesättigtes oder teilweise ungesättigtes Ringsystem mit 5-12 Ringgliedern bildet,

$R^7$ und $R^8$ unabhängig voneinander Wasserstoff, Hydroxy, Amino, Fluor, Amino-($C_1$-$C_4$)-alkyl, Hydroxy-($C_1$-$C_4$)-alkyl, ($C_1$-$C_4$)-Alkyl, das auch einfach ungesättigt sein kann, bedeuten,

$R'^0$ und $R'^1$ unabhängig voneinander Wasserstoff, Hydroxy oder ($C_6$-$C_{14}$)-Aryl bedeuten, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino, ($C_1$-$C_7$)-Alkylanino, Di-($C_1$-$C_7$)-alkylamino, Carboxy, Carboxymethoxy, Amino-($C_1$-$C_7$)-alkyl, ($C_1$-$C_7$)-Alkylamino-($C_1$-$C_7$)-alkyl, Di-($C_1$-$C_7$)-alkylamino-($C_1$-$C_7$)-alkyl, ($C_1$-$C_7$)-Alkoxycarbonylmethoxy, Carbamoyl, Sulfamoyl, ($C_1$-$C_7$)-Alkoxysulfonyl, Sulfo-und Guanidino-($C_1$-$C_8$)alkyl substituiert ist, oder für den Rest eines 5- oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben ($C_6$-$C_{14}$)-Aryl mono- oder disubstituiert ist,

n und m unabhängig voneinander 0, 1, 2, 3 und 4 sein können,

$R'^2$ Wasserstoff oder ($C_1$-$C_8$)-Alkyl ist, oder gemeinsam mit $R'$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,

$A^2$ entweder abwesend ist, wobei gleichzeitig A' nicht für einen Rest der Formel II steht, oder einen N-terminal mit A' und C-terminal mit dem Aminoboronsäurederivat verknüpften Rest der Formel VIII bedeutet,

$$\begin{array}{ccccc} R^6 & & R^5 & & O \\ | & & | & & \| \\ -\ N & - & CH & - & C\ - \end{array} \qquad (VIII)$$

wobei $R^5$ und $R^6$ wie oben definiert sind,

$R'^3$ Wasserstoff oder ($C_1$-$C_{12}$)-Alkyl, das gegebenenfalls ein- bis zweifach ungesättigt ist und das gegebenenfalls durch bis zu 3 gleiche oder verschiedene Reste aus der Reihe Hydroxy, ($C_1$-$C_7$)-Alkoxy, Amino, Mono- oder Di-($C_1$-$C_7$)-alkylamino substituiert ist, bedeutet

$R'^4$ und $R'^5$ unabhängig voneinander Wasserstoff, ($C_1$-$C_8$)-Alkyl, Hydroxymethyl, 2-Hydroxyethyl, (3-Hydroxysulfonyl,2-hydroxypropyl)amino, (2-Hydroxysulfonylethyl)amino, (2-Hydroxysulfonylpropyl)amino, (Carboxylmethyl)amino, Bis(2-hydroxyethyl)amino bedeuten sowie deren physiologisch verträglichen Salzen, zur Therapie und Prophylaxe von Viruserkrankungen, insbesondere von Erkrankungen, die durch das HIV verursacht werden.

2. Verwendung von Verbindungen der Formel I gemäß Anspruch 1, in welcher

$A'$ wie in Anspruch 1 definiert ist,

$R^1$ Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$-alkyl, gegebenenfalls substituiertes Phenyl-($C_1$-$C_8$)-alkyl, 2-Pyridyl-($C_1$-$C_8$)-alkyl, 3-

Pyridyl-($C_1$-$C_8$)-alkyl, 4-Pyridyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl,($C_1$-$C_4$)-Alkoxycarbonyl-($C_1$-$C_{10}$)-alkyl, ($C_1$-$C_8$)-Alkylsulfonyl, ($C_1$-$C_8$)-Alkylsulfinyl, Hydroxy-($C_1$-$C_{10}$)-alkanoyl, ($C_1$-$C_8$)-Alkanoyloxy-($C_1$-$C_{10}$)-alkyl, ($C_1$-$C_{11}$)-Alkanoyl, gegebenenfalls geschütztes Amino-($C_1$-$C_{11}$)-alkanoyl, Di-($C_1$-$C_7$)-alkylamino-($C_2$-$C_{11}$)-alkanoyl, ($C_3$-$C_9$)-Cycloalkylcarbonyl, ($C_6$-$C_{10}$)-Aryl-($C_2$-$C_{11}$)-alkanoyl, 2-Pyridyl-($C_1$-$C_8$)-alkanoyl, 3-Pyridyl-($C_1$-$C_8$)-alkanoyl, 4-Pyridyl-($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, Pyrrolyl-2-carbonyl, Pyridyl-3-carbonyl, Benzolsulfonyl, ($C_1$-$C_{10}$)-Alkoxycarbonyl, substituiertes ($C_1$-$C_{10}$)-Alkoxycarbonyl, ($C_6$-$C_{14}$)-Aryl-($C_1$-$C_6$)-alkoxycarbonyl, steht, oder gemeinsam mit $R^{12}$ ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxydiert sein kann,

$R^2$ ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, Dithiolanyl, Dithiolanylmethyl, Dithiolanylethyl, Dithianyl, Dithianylmethyl oder Dithianylethyl ist

$R^3$ und $R^4$ unabhängig voneinander Wasserstoff oder ($C_1$-$C_{12}$)-Alkyl bedeuten oder zusammen mit Bor, X und Y ein mono-, bi- oder tricyclisches, gesättigtes oder teilweise ungesättigtes, gegebenenfalls mono-, di-, tri- oder tetra-($C_1$-$C_{12}$)-alkyliertes Ringsystem mit 5-18 Ringgliedern bilden, das außer Bor, X, Y und Kohlenstoff noch ein -O- Glied, ein -$NR^{13}$-Glied oder -$CR^{14}R^{15}$-Glied enthalten kann,

X und Y unabhängig voneinander -O- oder -$NR^{13}$- sind,

$R^5$ und $R^9$ unabhängig voneinander für ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Carbamoyl, Guanidino, ($C_1$-$C_5$)-Alkoxycarbonylamino, ($C_7$-$C_{15}$)-Aralkoxycarbonylamino und 9-Fluorenylmethoxycarbonylamino substituiert ist, ($C_3$-$C_{12}$)-Cycloalkyl-($C_1$-$C_3$)-alkyl, mono- oder bicyclisches ($C_6$-$C_{14}$)-Aryl-($C_1$-$C_3$)-Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist oder für ($C_1$-$C_3$)-Alkyl, substituiert mit dem Rest eines 5- oder 6-gliedrigen monocyclischen oder 9-oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für ($C_6$-$C_{14}$)-Aryl beschrieben mono- oder disubstituiert ist, stehen,

$R^6$ Wasserstoff, Methyl oder Ethyl ist oder zusammen mit $R^5$ Pyrrolidin oder Piperidin bildet, die jeweils noch mit Cyclopentyl, Cyclohexyl oder Phenyl anneliert sein können,

$R^7$ und $R^8$ unabhängig voneinander Wasserstoff, Hydroxy, Amino, Fluor, Hydroxymethyl oder Aminomethyl sind,

$R^{10}$ und $R^{11}$ unabhängig voneinander Wasserstoff, Hydroxy, Phenyl, 2- oder 3-Thienyl, 2-, 3- oder 4-Pyridyl, 1-, 2- oder 4-Imidazolyl, 1- oder 2-Naphthyl; 2- oder 3-Benzo[b]thienyl sind,

n und m unabhängig voneinander 0, 1, 2, 3 und 4 bedeuten,

$R^{12}$ wie in Anspruch 1 definiert ist,

$R^{13}$ Wasserstoff oder ($C_1$-$C_{12}$)-Alkyl ist,

$R^{14}$ und $R^{15}$ wie in Anspruch 1 definiert sind,

$A^2$ abwesend ist, wobei gleichzeitig $A^1$ nicht für einen Rest der Formel II steht, oder einen Rest der Formel VIII bedeutet, wobei $R^5$ und $R^6$ wie in Anspruch 1 definiert sind,

sowie deren physiologisch verträglichen Salzen.

3. Verwendung von Verbindungen der Formel I, gemäß einem oder mehreren der Ansprüche 1 bis 2 in welcher,

$A^1$ wie in Anspruch 1 definiert ist,

$R^1$ ($C_1$-$C_8$)-Alkylsulfonyl, ($C_1$-$C_8$)-Alkylsulfinyl, Hydroxy-($C_1$-$C_{10}$)alkanoyl, ($C_1$-$C_8$)-Alkanoyloxy-($C_1$-$C_{10}$)-alkyl, ($C_1$-$C_{11}$)Alkanoyl, Amino-($C_1$-$C_{11}$)-alkanoyl, Di-($C_1$-$C_7$)-alkylamino-($C_2$-$C_{11}$)-alkanoyl, ($C_3$-$C_9$)-Cycloalkylcarbonyl, ($C_6$-$C_{10}$)-Aryl-($C_2$-$C_{11}$)-alkanoyl, 2-Pyridyl-($C_1$-$C_8$)alkanoyl, 3-Pyridyl-($C_1$-$C_8$)-alkanoyl, 4-Pyridyl($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, Pyrrolyl 2-carbonyl, Pyridyl-3-carbonyl, Benzolsulfonyl, ($C_1$-$C_{10}$)-Alkoxycarbonyl, substituiertes ($C_1$-$C_{10}$)-Alkoxycarbonyl, ($C_6$-$C_{14}$)-Aryl-($C_1$-$C_6$)-alkoxycarbonyl bedeutet

$R^2$ ($C_3$-$C_{12}$)-Alkyl, mono-, bi- oder tricyclisches ($C_3$-$C_{18}$)-Cycloalkyl oder ($C_3$-$C_{18}$)-Cycloalkylmethyl wobei der Cycloalkylteil gegebenenfalls durch ($C_1$-$C_4$)-Alkyl substituiert ist, ($C_6$-$C_{14}$)-Arylmethyl, Dithiolanyl, Dithiolanylmethyl, Dithianyl und Dithianylmethyl ist,

$R^3$ und $R^4$ Wasserstoff sind oder gemeinsam mit Bor, X und Y ein mono-, bi- oder tricyclisches mono-, di-, tri-oder tetra-($C_1$-$C_{12}$)alkyliertes, gesättigtes oder teilweise ungesättigtes Ringsystem mit 5-18 Ringgliedern bilden, das außer Bor, X, Y und Kohlenstoff noch ein -O- Glied,ein -$NR^{13}$-Glied oder ein -$CR^{14}R^{15}$-Glied

enthalten kann,
X und Y für -O- stehen,
$R^5$ und $R^9$ unabhängig voneinander Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto, 1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)-ethyl, 3-Thienyl, 3-Thienylmethyl, 2-(3-Thienyl)ethyl, 4-Chlorbenzyl, 2-(Methylsulfinyl)ethyl, 2-(Methylsulfonyl)methyl, 2-Pyridylmethyl, 3-Pyridylmethyl, 4-Pyridylmethyl, Cyclohexyl, (1-Methyl-imidazol-4-yl)methyl, (3-Methyl-Imidazol-4-yl)methyl, Phenyl, 1-Naphthylmethyl, 2-Naphthylmethyl, 2-Phenyl-ethyl, 2-Thiazolylmethyl, 4-Thiazolylmethyl, 3-Pyrazolylmethyl, 4-Pyrimidinylmethyl, Indol-2-yl-methyl, 2-Benzo[b]-thienylmethyl, 3-Benzo[b]thienylmethyl, 2-Furylmethyl, Cyclopentyl sind,
$R^6$ Wasserstoff oder Methyl bedeutet oder gemeinsam mit $R^5$ und der diese Reste tragenden -N-CH-Gruppe ein Tetrahydroisochinolin- oder Azabicyclooctan-Gerüst bildet,
einer der Reste $R^7$ oder $R^8$ Wass rstoff bedeutet, der jeweils andere Rest Hydroxy, Amino, Fluor, Hydroxymethyl oder Aminomethyl,
$R^{10}$ und $R^{11}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,
n und m unabhängig voneinander 0, 1 oder 2 sind,
$R^{12}$ Wasserstoff oder Methyl ist, oder gemeinsam mit $R^1$ ein mono- oder bicyclisches gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann,
$R^{13}$ Wasserstoff oder $(C_1-C_4)$-Alkyl ist,
$R^{14}$ und $R^{15}$ wie in Anspruch 1 definiert sind, $A^2$ abwesend ist, wobei gleichzeitig $A^1$ nicht für einen Rest der Formel II steht, oder einen Rest der Formel VIII bedeutet, wobei $R^5$ und $R^6$ wi in Anspruch 2 definiert sind,
sowie deren physiologisch verträglichen Salzen.

4. Verwendung von Verbindungen der Formel I, gemäß einem oder mehreren der Ansprüche 1-3, in welchen
$A^1$ für einen Rest de r Formel II, III oder VI steht, worin
$R^1$ $(C_1-C_6)$-Alkylsulfonyl; $(C_1-C_6)$-Alkylsulfinyl; Amino-$(C_1-C_6)$-alkanoyl; $(C_1-C_6)$-Alkoxycarbonyl; $(C_6-C_{10})$-Aryl-$(C_1-C_5)$-alkoxycarbonyl bedeutet,
$R^2$ $(C_5-C_8)$-Cycloalkyl; $(C_5-C_{11})$-Cycloalkylmethyl; [$(C_1-C_4)$-Alkyl-cyclohexyl]methyl; $(C_3-C_4)$-Alkyl, das durch $(C_1-C_3)$-Alkyl substituiert ist; $(C_6-C_{10})$-Arylmethyl oder Dithiolan-2-yl-methyl bedeutet,
$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2′-iminodiethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,
X und Y für -O- stehen,
$R^5$ Wasserstoff, n-Propyl, Isopropyl, n-Butyl, Isobutyl, Cyclopentyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,
$R^6$ für Wasserstoff oder Methyl steht,
$R^{10}$ und $R^{11}$ unabhängig voneinander 1-Naphthyl oder 2-Thienyl bedeuten,
n und m für 1 stehen,
$R^{12}$ Wasserstoff bedeutet,
$R^{14}$ und $R^{15}$ unabhängig voneinander Wasserstoff, $(C_1-C_8)$-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)-amino, (Carboxymethyl) amino oder Bis(2-hydroxyethyl)-amino bedeuten,
und
$A^2$ für einen Rest der Formel VIII steht, wobei $R^5$ und $R^6$ wie in Anspruch 3 definiert sind,
sowie deren physiologisch verträglichen Salzen.

5. Verwendung von Verbindungen der Formel I gemäß einem oder mehreren der Ansprüche 1-4 zur Herstellung eines Arzneimittels zur Therapie oder Prophylaxe von Viruserkrankungen, insbesondere von Erkrankungen die durch das HIV verursacht werden.

6. Arzneimittel zur Therapie und Prophylaxe von Viruserkrankungen, insbesondere von Erkrankungen die durch das HIV verursacht werden, dadurch gekennzeichnet, daß es mindestens eine Verbindung der Formel I gemäß einem oder mehreren der Ansprüche 1-4 enthält.

7. Verfahren zur Herstellung von Arzneimitteln zur Therapie und Prophylaxe von Viruserkrankungen, insbesondere von Erkrankungen, die durch das HIV verursacht werden, dadurch gekennzeichnet, daß mindestens eine Verbindung der Formel I gemäß einem oder mehreren der Ansprüche 1-4 mit geeigneten Trägerstoffen in eine geeignete Darreichungsform gebracht wird.

8. Verfahren zur Therapie und Prophylaxe von Viruserkrankungen, insbesondere von Erkrankungen die durch das HIV verursacht werden, dadurch gekennzeichnet, daß man eine Verbindung der Formel I gemäß einem oder mehreren der Ansprüche 1-4 verabreicht.

Patentansprüche für folgende Vertragsstaaten: ES; GR

1. Verwendung von Verbindung der Formel I

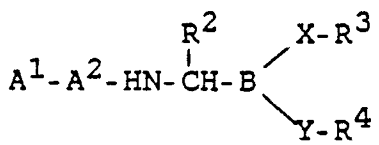

(I)

in welcher
$A^1$ einen Rest der Formeln II, III, IV, V oder VI bedeutet

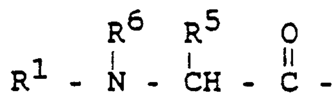

(II)

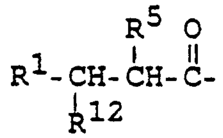

(III)

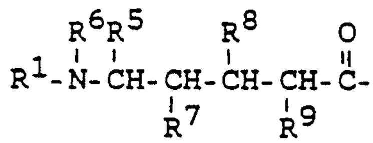

(IV)

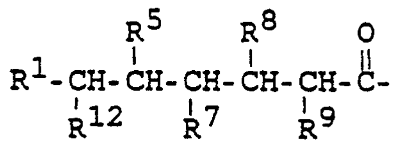

(V)

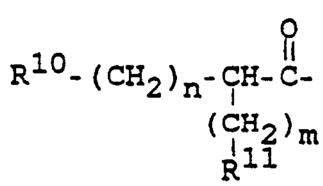

(VI)

worin
$R^1$ $a_1$) Wasserstoff, $(C_1-C_{12})$-Alkyl, das gegebenenfalls ein- oder zweifach ungesättigt ist und das gegebenenfalls durch bis zu 3 gleiche oder verschiedene Reste aus der Reihe Hydroxy, $(C_1-C_7)$-Alkoxy, $(C_1-C_8)$-Alkanoyloxy, Carboxy, $(C_1-C_7)$-Alkoxycarbonyl, F, Cl, Br, I, Amino, Carbamoyl, Amidino, das gegebenenfalls durch einen, zwei oder drei $(C_1-C_8)$-Alkylreste substituiert sein kann, Guanidino, das gegebenenfalls durch

einen, zwei, drei oder vier $(C_1-C_8)$-Alkylreste substituiert sein kann, $(C_1-C_7)$-Alkylamino, Di-$(C_1-C_7)$-alkylamino, $(C_1-C_5)$-Alkoxycarbonylamino, $(C_7-C_{15})$-Aralkoxycarbonylamino und 9-Fluorenylmethoxycarbonylamino substituiert ist, mono-, bi- oder tricyclisches $(C_3-C_{18})$-Cycloalkyl, $(C_3-C_{18})$-Cycloalkyl-$(C_1-C_6)$-alkyl oder $(C_6-C_{14})$-Aryl, das gegebenenfalls durch einen oder zwei gleiche oder verschiedene Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino, gegebenenfalls mit bis zu 2 Halogenen substituiertes Anilino und Trifluormethyl substituiert ist, $(C_6-C_{14})$-Aryl-$(C_1-C_6)$-alkyl, worin der Arylteil gegebenenfalls durch einen oder zwei gleiche oder verschiedene Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo- und Guanidino-$(C_1-C_8)$-alkyl substituiert ist, oder für den Rest eines 5- oder 6-gliedrigen monocyclischen oder 9- oder 10-gliedrigen bicyclischen teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder steht,und der gegebenenfalls wie $(C_6-C_{14})$-Aryl unter $a_1$) definiert, mono-, di- oder trisubstituiert ist, bedeutet

oder

$a_2$) einen Rest der Formel VII bedeutet

$R^1$ - W , (VII)

worin $R^1$ wie $R^1$ unter $a_1$) definiert ist und W für -CO-, -O-CO-, $-SO_2-$, -SO-, $-HN-SO_2-$, -HN-CO-, -CH(OH)- oder -N(OH)- steht,

$R^2$, $R^5$ und $R^9$ unabhängig voneinander wie $R^1$ unter $a_1$) definiert sind,

$R^3$ und $R^4$ unabhängig voneinander Wasserstoff oder $(C_1-C_{12})$-Alkyl bedeuten, das gegebenenfalls ein- bis zweifach ungesättigt ist und das gegebenenfalls durch bis zu 3 gleiche oder verschiedene Reste aus der Reihe Hydroxy, $(C_1-C_7)$-Alkoxy, Amino, Mono- oder Di-$(C_1-C_7)$-alkylamino substituiert ist, oder zusammen mit Bor, X und Y ein mono-, bi- oder tricyclisches, gesättigtes oder teilweise ungesättigtes mono-, di-, tri- oder tetra-$(C_1-C_{12})$-alkyliertes Ringsystem mit 5-18 Ringgliedern, das außer Bor, X, Y und Kohlenstoff noch ein -O- Glied, ein $-NR^{13}$-Glied oder ein $-CR^{14}R^{15}$-Glied enthalten kann, bilden,

X und Y unabhängig voneinander für -O- oder $-NR^{13}$-stehen,

$R^6$ für Wasserstoff oder $(C_1-C_8)$-Alkyl steht, oder zusammen mit $R^5$ ein mono- oder bicyclisches, gesättigtes oder teilweise ungesättigtes Ringsystem mit 5-12 Ringgliedern bildet,

$R^7$ und $R^8$ unabhängig voneinander Wasserstoff, Hydroxy, Amino, Fluor, Amino-$(C_1-C_4)$-alkyl, Hydroxy-$(C_1-C_4)$-alkyl, $(C_1-C_4)$-Alkyl, das auch einfach ungesättigt sein kann, bedeuten,

$R^{10}$ und $R^{11}$ unabhängig voneinander Wasserstoff, Hydroxy oder $(C_6-C_{14})$-Aryl bedeuten, das gegebenenfalls durch einen oder zwei gleiche oder verschiedene Reste aus der Reihe F, Cl, Br, I, Hydroxy, $(C_1-C_7)$-Alkoxy, $(C_1-C_7)$-Alkyl, $(C_1-C_7)$-Alkoxycarbonyl, Amino, $(C_1-C_7)$-Alkylamino, Di-$(C_1-C_7)$-alkylamino, Carboxy, Carboxymethoxy, Amino-$(C_1-C_7)$-alkyl, $(C_1-C_7)$-Alkylamino-$(C_1-C_7)$-alkyl, Di-$(C_1-C_7)$-alkylamino-$(C_1-C_7)$-alkyl, $(C_1-C_7)$-Alkoxycarbonylmethoxy, Carbamoyl, Sulfamoyl, $(C_1-C_7)$-Alkoxysulfonyl, Sulfo-und Guanidino-$(C_1-C_8)$alkyl substituiert ist, oder für den Rest eines 5- oder 6-gliedrigen monocyclischen oder 9- oder 10-gliedrigen bicyclischen, gegebenenfalls teilweise oder vollständig hydrierten Heteroaromaten, mit mindestens 1 C-Atom, 1 - 4 N-Atomen und/oder 1 - 2 S-Atomen und/oder 1 - 2 O-Atomen als Ringglieder stehen, der gegebenenfalls wie oben $(C_6-C_{14})$-Aryl mono- oder di substituiert ist, 1

n und m unabhängig voneinander 0, 1, 2, 3 und 4 sein können,

$R^{12}$ Wasserstoff oder $(C_1-C_8)$-Alkyl ist, oder gemeinsam mit $R^1$ ein mono- oder bicyclisches, gesättigtes oder teilweise ungesättigtes Ringsystem mit 5 - 12 Ringgliedern bildet, das außer Kohlenstoff noch 1 Schwefelatom enthalten kann, welches gegebenenfalls zum Sulfoxid oder Sulfon oxidiert sein kann,

$A^2$ entweder abwesend ist, wobei gleichzeitig $A^1$ nicht für einen Rest der Formel II steht, oder einen N-terminal mit $A^1$ und C-terminal mit dem Aminoboronsäurederivat verknüpften Rest der Formel VIII bedeutet,

$$-\overset{R^6}{\overset{|}{N}}-\overset{R^5}{\overset{|}{CH}}-\overset{O}{\overset{\|}{C}}- \qquad (VIII)$$

wobei $R^5$ und $R^6$ wie oben definiert sind,

$R^{13}$ Wasserstoff oder $(C_1-C_{12})$-Alkyl, das gegebenenfalls ein- bis zweifach ungesättigt ist und das gegebenenfalls durch bis zu 3 gleiche oder verschiedene Reste aus der Reihe Hydroxy, $(C_1-C_7)$-Alkoxy, Amino, Mono- oder Di-$(C_1-C_7)$-alkylamino substituiert ist, bedeutet

$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl,2-hydroxypropyl)amino, (2-Hydroxysulfonylethyl)amino, (2-Hydroxysulfonylpropyl)amino, (Carboxylmethyl)amino, Bis(2-hydroxyethyl)amino bedeuten sowie deren physiologisch verträglichen Salzen, zur Therapie und Prophylaxe von Viruserkrankungen, insbesondere von Erkrankungen, die durch das HIV verursacht werden.

2. Verwendung von Verbindungen der Formel I gemäß Anspruch 1, in welcher

$A^1$ wie in Anspruch 1 definiert ist,

$R^1$ Wasserstoff bedeutet oder für ($C_1$-$C_{10}$)-Alkyl, Cyclopentyl, Cyclohexyl, Cyclopentyl-($C_1$-$C_{10}$)-alkyl, Cyclohexyl-($C_1$-$C_{10}$)-alkyl, gegebenenfalls substituiertes Phenyl-($C_1$-$C_8$)-alkyl, 2-Pyridyl-($C_1$-$C_8$)-alkyl, 3-Pyridyl-($C_1$-$C_8$)-alkyl, 4-Pyridyl-($C_1$-$C_8$)-alkyl, $H_2N$-($C_1$-$C_{10}$)-Alkyl, HO-($C_1$-$C_{10}$)-Alkyl, ($C_1$-$C_4$)-Alkoxy-($C_1$-$C_{10}$)-alkyl,($C_1$-$C_4$)-Alkoxycarbonyl-($C_1$-$C_{10}$)-alkyl, ($C_1$-$C_8$)-Alkylsulfonyl, ($C_1$-$C_8$)-Alkylsulfinyl. Hydroxy-($C_1$-$C_{10}$)-alkanoyl, ($C_1$-$C_8$)-Alkanoyloxy-($C_1$-$C_{10}$)-alkyl, ($C_1$-$C_{11}$)-Alkanoyl, gegebenenfalls geschütztes Amino-($C_1$-$C_{11}$)-alkanoyl, Di-($C_1$-$C_7$)-alkylamino-($C_2$-$C_{11}$)-alkanoyl, ($C_3$-$C_9$)-Cycloalkylcarbonyl, ($C_6$-$C_{10}$)-Aryl-($C_2$-$C_{11}$)-alkanoyl, 2-Pyridyl-($C_1$-$C_8$)-alkanoyl, 3-Pyridyl-($C_1$-$C_8$)-alkanoyl, 4-pyridyl-($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxycarbonyl substituiertes Benzoyl, Pyrrolyl-2-carbonyl, Pyridyl-3-carbonyl, Benzolsulfonyl, ($C_1$-$C_{10}$)-Alkoxycarbonyl, substituiertes ($C_1$-$C_{10}$)-Alkoxycarbonyl, ($C_6$-$C_{14}$)-Aryl-($C_1$-$C_6$)-alkoxycarbonyl, steht, oder gemeinsam mit $R^{12}$ ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxydiert sein kann,

$R^2$ ($C_3$-$C_{12}$)-Alkyl, mono-, bi- oder tricyclisches ($C_3$-$C_{18}$)-Cycloalkyl, ($C_3$-$C_{18}$)-Cycloalkylmethyl, ($C_3$-$C_{18}$)-Cycloalkylethyl, Dithiolanyl, Dithiolanylmethyl, Dithiolanylethyl, Dithianyl, Dithianylmethyl oder Dithianylethyl ist

$R^3$ und $R^4$ unabhängig voneinander Wasserstoff oder ($C_1$-$C_{12}$)-Alkyl bedeuten oder zusammen mit Bor, X und Y ein mono-, bi- oder tricyclisches, gesättigtes oder teilweise ungesättigtes, gegebenenfalls mono-, di-, tri- oder tetra-($C_1$-$C_{12}$)-alkyliertes Ringsystem mit 5-18 Ringgliedern bilden, das außer Bor, X, Y und Kohlenstoff noch ein -O- Glied, ein -$NR^{13}$-Glied oder -$CR^{14}R^{15}$-Glied enthalten kann, 1

X und Y unabhängig voneinander -O- oder -$NR^{13}$- sind, $R^5$ und $R^9$ unabhängig voneinander für ($C_1$-$C_{10}$)-Alkyl, das gegebenenfalls ein- oder zweifach ungesättigt ist und das gegebenenfalls durch bis zu 3 gleiche oder verschiedene Reste aus der Reihe Hydroxy, ($C_1$-$C_7$)-Alkoxy, ($C_1$-$C_7$)-Alkanoyloxy, Carboxy, ($C_1$-$C_7$)-Alkoxycarbonyl, Cl, Br, Amino, Amidino, Carbamoyl, Guanidino, ($C_1$-$C_5$)-Alkoxycarbonylamino, ($C_7$-$C_{15}$)-Aralkoxycarbonylamino und 9-Fluorenylmethoxycarbonylamino substituiert ist, ($C_3$-$C_{12}$)-Cycloalkyl-($C_1$-$C_3$)-alkyl, mono- oder bicyclisches ($C_6$-$C_{14}$)-Aryl-($C_1$-$C_3$)Alkyl, das gegebenenfalls durch einen oder zwei gleiche oder verschiedene Reste aus der Reihe F, Cl, Br, I, Hydroxy, ($C_1$-$C_7$)-Alkoxy, ($C_1$-$C_7$)-Alkyl, ($C_1$-$C_7$)-Alkoxycarbonyl, Amino und Trifluormethyl substituiert ist oder für ($C_1$-$C_3$)-Alkyl, substituiert mit dem Rest eines 5- oder 6-gliedrigen monocyclischen oder 9-oder 10-gliedrigen bicyclischen, gegebenenfalls teilweise oder vollständig hydrierten Heteroatomen, mit mindestens 1 C-Atom, 1 - 4 N-Atomen und/oder 1 - 2 S-Atomen und/oder 1 - 2 O-Atomen als Ringglieder, der gegebenenfalls wie in Anspruch 1 für ($C_6$-$C_{14}$)-Aryl beschrieben mono- oder disubstituiert ist, stehen,

$R^6$ Wasserstoff, Methyl oder Ethyl ist oder zusammen mit $R^5$ Pyrrolidin oder Piperidin bildet, die jeweils noch mit Cyclopentyl, Cyclohexyl oder Phenyl anneliert sein können,

$R^7$ und $R^8$ unabhängig voneinander Wasserstoff, Hydroxy, Amino, Fluor, Hydroxymethyl oder Aminomethyl sind,

$R^{10}$ und $R^{11}$ unabhängig voneinander Wasserstoff, Hydroxy, Phenyl, 2- oder 3-Thienyl, 2-, 3- oder 4-Pyridyl, 1-, 2- oder 4-imidazolyl, 1- oder 2-Naphthyl; 2- oder 3-Benzo[b]thienyl sind,

n und m unabhängig voneinander 0, 1, 2, 3 und 4 bedeuten,

$R^{12}$ wie in Anspruch 1 definiert ist,

$R^{13}$ Wasserstoff oder ($C_1$-$C_{12}$)-Alkyl ist,

$R^{14}$ und $R^{15}$ wie in Anspruch 1 definiert sind, $A^2$ abwesend ist, wobei gleichzeitig $A^1$ nicht für einen Rest der Formel II steht, oder einen Rest der Formel VIII bedeutet, wobei $R^5$ und $R^6$ wie in Anspruch 1 definiert sind, sowie deren physiologisch verträglichen Salzen.

3. Verwendung von Verbindungen der Formel I, gemäß einem oder mehreren der Ansprüche 1 bis 2 in welcher,

$A^1$ wie in Anspruch 1 definiert ist,

$R^1$ ($C_1$-$C_8$)-Alkylsulfonyl, ($C_1$-$C_8$)-Alkylsulfinyl, Hydroxy-($C_1$-$C_{10}$)alkanoyl, ($C_1$-$C_8$)-Alkanoyloxy-($C_1$-$C_{10}$)-alkyl, ($C_1$-$C_{11}$)Alkanoyl, Amino-($C_1$-$C_{11}$)-alkanoyl, Di-($C_1$-$C_7$)-alkylamino-($C_2$-$C_{11}$)-alkanoyl, ($C_3$-$C_9$)-Cycloalkylcarbonyl, ($C_6$-$C_{10}$)-Aryl-($C_2$-$C_{11}$)-alkanoyl, 2-Pyridyl-($C_1$-$C_8$)alkanoyl, 3-Pyridyl-($C_1$-$C_8$)-alkanoyl, 4-Pyridyl($C_1$-$C_8$)-alkanoyl, gegebenenfalls durch Halogen, ($C_1$-$C_7$)-Alkyl, ($C_1$-$C_7$)-Alkoxy oder ($C_1$-$C_7$)-Alkoxyc-

arbonyl substituiertes Benzoyl, Pyrrolyl-2-carbonyl, Pyridyl-3-carbonyl, Benzolsulfonyl, ($C_1$-$C_{10}$)-Alkoxycarbonyl, substituiertes ($C_1$-$C_{10}$)-Alkoxycarbonyl, ($C_6$-$C_{14}$)-Aryl-($C_1$-$C_6$)-alkoxycarbonyl bedeutet

$R^2$ ($C_3$-$C_{12}$)-Alkyl, mono-, bi- oder tricyclisches ($C_3$-$C_{18}$)-Cycloalkyl oder ($C_3$-$C_{18}$)-Cycloalkylmethyl wobei der Cycloalkylteil gegebenenfalls durch ($C_1$-$C_4$)-Alkyl substituiert ist, ($C_6$-$C_{14}$)-Arylmethyl, Dithiolanyl, Dithiolanylmethyl, Dithianyl und Dithianylmethyl ist,

$R^3$ und $R^4$ Wasserstoff sind oder gemeinsam mit Bor, X und Y ein mono-, bi- oder tricyclisches mono-, di-, tri-oder tetra-($C_1$-$C_{12}$)alkyliertes, gesättigtes oder teilweise ungesättigtes Ringsystem mit 5-18 Ringgliedern bilden, das außer Bor, X, Y und Kohlenstoff noch ein -O- Glied,ein -$NR^{13}$-Glied oder ein -$CR^{14}R^{15}$-Glied enthalten kann,

X und Y für -O- stehen,

$R^5$ und $R^9$ unabhängig voneinander Methyl, Ethyl, Isopropyl, n-Propyl, n-Butyl, Isobutyl, sec.-Butyl, 3-Guanidinopropyl, Carbamoylmethyl, 2-Carbamoylethyl, Carboxymethyl, 2-Carboxyethyl, Mercaptomethyl, 2-(Methylthio)ethyl, (1-Mercapto, 1-methyl)ethyl, Hydroxymethyl, 1-Hydroxyethyl, Amino, Aminomethyl, 2-Aminoethyl, 3-Aminopropyl, 4-Aminobutyl, N,N-Dimethylamino, Cyclohexylmethyl, Imidazol-4-yl-methyl, Benzyl, 2-Methylbenzyl, 3-Methylbenzyl, Indol-3-yl-methyl, 4-Hydroxybenzyl, 4-Methoxybenzyl, 3,4-Dihydroxybenzyl, 3,4-Dimethoxybenzyl, (Benzdioxolan-5-yl)methyl, 2-Thienyl, 2-Thienylmethyl, 2-(2-Thienyl)-methyl, 3-Thienyl, 3-Thienylmethyl, 2-(3-Thienyl)ethyl, 4-Chlorbenzyl, 2-(Methylsulfinyl)ethyl, 2-(Methylsulfonyl)ethyl, 2-Pyridylmethyl, 3-Pyridylmethyl, 4-Pyridylmethyl, Cyclohexyl, (1-Methyl-imidazol-4-yl)methyl, (3-Methyl-Imidazol-4-yl)methyl, Phenyl, 1-Naphthylmethyl, 2-Naphthylmethyl, 2-Phenyl-ethyl, 2-Thiazolylmethyl, 4-Thiazolylmethyl, 3-Pyrazolylmethyl, 4-Pyrimidinylmethyl, Indol-2-yl-methyl, 2-Benzo[b]-thienylmethyl, 3-Benzo[b]thienylmethyl, 2-Furylmethyl, Cyclopentyl sind,

$R^6$ Wasserstoff oder Methyl bedeutet oder gemeinsam mit $R^5$ und der diese Reste tragenden -N-CH-Gruppe ein Tetrahydroisochinolin- oder Azabicyclooctan-Gerüst bildet,

einer der Reste $R^7$ oder $R^8$ Wasserstoff bedeutet, der jeweils andere Rest Hydroxy, Amino, Fluor, Hydroxymethyl oder Aminomethyl,

$R^{10}$ und $R^{11}$ unabhängig voneinander Wasserstoff, Hydroxy, Phenyl, 2-Thienyl, 2-, 3- oder 4-Pyridyl, 1- oder 2-Imidazolyl, 1-Naphthyl, 2- oder 3-Benzo[b]thienyl sind,

n und m unabhängig voneinander 0, 1 oder 2 sind,

$R^{12}$ Wasserstoff oder Methyl ist, oder gemeinsam mit $R^1$ ein mono- oder bicyclisches gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann,

$R^{13}$ Wasserstoff oder ($C_1$-$C_4$)-Alkyl ist,

$R^{14}$ und $R^{15}$ wie in Anspruch 1 definiert sind,

$A^2$ abwesend ist, wobei gleichzeitig $A^1$ nicht für einen Rest der Formel II steht, oder einen Rest der Formel VIII bedeutet, wobei $R^5$ und $R^6$ wie in Anspruch 2 definiert sind,

sowie deren physiologisch verträglichen Salzen.

4. Verwendung von Verbindungen der Formel I, gemäß einem oder mehreren der Ansprüche 1-3, in welchen

$A^1$ für einen Rest der Formel II, III oder VI steht, worin

$R^1$ ($C_1$-$C_6$)-Alkylsulfonyl; ($C_1$-$C_6$)-Alkylsulfinyl; Amino-($C_1$-$C_6$)-alkanoyl; ($C_1$-$C_6$)-Alkoxycarbonyl; ($C_6$-$C_{10}$)-Aryl-($C_1$-$C_6$)-alkoxycarbonyl bedeutet,

$R^2$ ($C_5$-$C_8$)-Cycloalkyl; ($C_5$-$C_{11}$)-Cycloalkylmethyl; [($C_1$-$C_4$)-Alkyl-cyclohexyl]methyl; ($C_3$-$C_4$)-Alkyl, das durch ($C_1$-$C_3$)-Alkyl substituiert ist; ($C_6$-$C_{10}$)-Arylmethyl oder Dithiolan-2-yl-methyl bedeutet,

$R^3$ und $R^4$ für Wasserstoff stehen oder zusammen mit Bor, X und Y einen 1,3,2-Dioxaborolan-Rest, der gegebenenfalls durch 1 bis 4 Methylgruppen substituiert ist, einen [4,5-Diisopropyl]-1,3,2-dioxaborolan-, einen [(N-B)-(2,2′-iminodiethanolato)-boryl)]-, einen Pinandioxyboryl- oder einen 1,3,2-Dioxaborinan-Rest, dessen C-Atom in Position 5 durch $R^{14}$ und $R^{15}$ substituiert ist, bilden,

X und Y für -O- stehen,

$R^5$ Wasserstoff, n-Propyl, Isopropyl, n-Butyl, Isobutyl, Cyclopentyl, sec.-Butyl, Carbamoylmethyl, 2-Carbamoylethyl, Benzyl, 2-Methylbenzyl, 3-Methylbenzyl, 2-Thienylmethyl, 3-Thienylmethyl, 1-Naphthylmethyl, 2-Naphthylmethyl, Imidazol-4-yl-methyl oder 2-Thiazolylmethyl bedeutet,

$R^6$ für Wasserstoff oder Methyl steht,

$R^{10}$ und $R^{11}$ unabhängig voneinander 1-Naphthyl oder 2-Thienyl bedeuten,

n und m für 1 stehen,

$R^{12}$ Wasserstoff bedeutet,

$R^{14}$ und $R^{15}$ unabhängig voneinander Wasserstoff, ($C_1$-$C_8$)-Alkyl, Hydroxymethyl, 2-Hydroxyethyl, (3-Hydroxysulfonyl, 2-hydroxypropyl)amino, (2-Hydroxysulfonylethyl)amino, (2-Hydroxysulfonylpropyl)-amino, (Carboxymethyl) amino oder Bis(2-hydroxyethyl)-amino bedeuten,

und
$A^2$ für einen Rest der Formel VIII steht, wobei $R^5$ und $R^6$ wie in Anspruch 3 definiert sind,
sowie deren physiologisch verträglichen Salzen.

5. Verwendung von Verbindungen der Formel I gemäß einem oder mehreren der Ansprüche 1-4 zur Herstellung eines Arzneimittels zur Therapie oder Prophylaxe von Viruserkrankungen, insbesondere von Erkrankungen die durch das HIV verursacht werden.

6. Verfahren zur Herstellung von Arzneimitteln zur Therapie und Prophylaxe von Viruserkrankungen, insbesondere von Erkrankungen, die durch das HIV verursacht werden, dadurch gekennzeichnet, daß mindestens eine Verbindung der Formel I gemäß einem oder mehreren der Ansprüche 1-4 mit geeigneten Trägerstoffen in eine geeignete Darreichungsform gebracht wird.

7. Verfahren zur Therapie und Prophylaxe von Viruserkrankungen, insbesondere von Erkrankungen die durch das HIV verursacht werden, dadurch gekennzeichnet, daß man eine Verbindung der Formel I gemäß einem oder mehreren der Ansprüche 1-4 verabreicht.

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

Nummer der Anmeldung

EP 89114601.1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,P, D | EP - A - 0 315 574 (HOECHST AG) * Seite 8, Zeilen 28-30 * | 1-7 | A61K37/64 C07F5/02 |
| X,P | EP - A - 0 293 881 (DU PONT DE NEMOURS) * Seite 1, Zeilen 15-27 * | 1-7 | |
| X | EP - A - 0 145 441 (DU PONT DE NEMOURS) * Seite 1 * | 1-7 | |
| A | US - A - 4 537 773 (A.B. SHENVI) * das ganze Dokument * | 1-7 | |
| A | CHEMICAL ABSTRACTS vol. 102, no. 6, 11.Februar 1985, Seite 260, Spalte 1, Zusammenfassung Nr. 58256d, Columbus, Ohio, USA; C.A. KETTNER et al.: "Inhibition of the serine proteases leukocyte elastase, pancreatic elastase, cathepsin G, and chymotrypsin by peptide boronic acids" &J. Biol.Chem. 1984, 259(24), 15106-14 | 1-7 | RECHERCHIERTE SACHGEBIETE (Int. Cl.4) C07F A61K |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-7

Unvollständig recherchierte Patentansprüche:

Nicht recherchierte Patentansprüche: 8

Grund für die Beschränkung der Recherche: Artikel 52(4) EPÜ

| Recherchenort | Abschlußdatum der Recherche | Prüfer |
|---|---|---|
| Berlin | 23.10.1989 | P.AVEDIKIAN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03 82